# EUROPÄISCHE PATENTSCHRIFT

(11) **EP 1 487 497 B1**
(45) Veröffentlichungstag und Bekanntmachung des Hinweises auf die Patenterteilung: **24.01.2007**
(21) Anmeldenummer: 03732201.3
(22) Anmeldetag: 19.03.2003
(51) Int. Cl.: A61K 47/18, A61K 39/39, G01N 33/50, A61K 35/14, G01N 33/52, G01N 33/53, G01N 33/68, G01N 33/569

(54) **VERWENDUNG HARNSTOFF-ADJUVIERTER POLYPEPTIDE ZUR DIAGNOSE, PROPHYLAXE UND THERAPIE**
USE OF UREA-ADJUVATED POLYPEPTIDES FOR DIAGNOSIS, PROPHYLAXIS AND TREATMENT
UTILISATION DE POLYPEPTIDES AJOUTES A DE L'UREE POUR LE DIAGNOSTIC, LA PREVENTION ET LA THERAPIE

(30) Priorität: 22.03.2002 DE 10212867
(43) Veröffentlichungstag der Anmeldung: 22.12.2004
(73) Patentinhaber: Wolf, Hans, 82319 Starnberg (DE)
(72) Erfinder: WOLF, Hans, 82319 Starnberg (DE); DEML, Ludwig, 93128 Regenstauf (DE); PÜLLMANN, Kerstin, 93047 Regensburg (DE)
(74) Vertreter: Dehmel, Albrecht
(86) Internationale Anmeldenummer: PCT/DE2003/000917
(87) Internationale Veröffentlichungsnummer: WO 2003/080792

(56) Entgegenhaltungen:
- GB-A- 1 128 005
- SCHIRMBECK R. ET AL: "Priming of class I-restricted cytotoxic T lymphocytes by vaccination with recombinant protein antigens" VACCINE, BUTTERWORTH SCIENTIFIC. GUILDFORD, GB, Bd. 13, Nr. 9, 1. Juni 1995 (1995-06-01), Seiten 857-865, XP004057542 ISSN: 0264-410X
- ELLIOTT S L ET AL: "DOMINANT CYTOTOXIC T LYMPHOCYTE RESPONSE TO THE IMMEDIATE-EARLY TRANS-ACTIVATOR PROTEIN, BZLF1, IN PERSISTENT TYPE A OR B EPSTEIN-BARR VIRUS INFECTION" JOURNAL OF INFECTIOUS DISEASES, CHICAGO, IL, US, Bd. 176, Oktober 1997 (1997-10), Seiten 1068-1072, XP000943486 ISSN: 0022-1899
- O'HAGAN D.T. ET AL.: "Recent developments in adjuvants for vaccines against infectious diseases" BIOMOL. ENGIN., Bd. 18, 2001, Seiten 69-85, XP002256565
- B. BAUER ET AL.: "Induction of MHC class-I and -II restricted epitope presentation by urea-treated BZLF1 protein: a novel technology for the detection of protein-specific cytotoxic T-cell" PROCEEDINGS OF THE EBV2002 CONFERENCE, 16. - 20. Juli 2002, Seite 34 XP002256566 Cairns,Australia

### SEQUENCE LISTING

<110> WOLF, Hans
<120> VERWENDUNG HARNSTOFF-ADJUVIERTER POLYPEPTIDE ZUR DIAGNOSE, PROPHYLAXE UND THERAPIE
<130> URD-001 PCT
<150> DE 102 12 867.7
<151> 2002-03-22
<160> 3
<170> PatentIn version 3.1
<210> 1
   <211> 30
   <212> DNA
   <213> artificial sequence
<220>
   <223> Primer
<400> 1
   ggcggagatc tttagaaatt taagagatcc 30
<210> 2
   <211> 30
   <212> DNA
   <213> artificial sequence
<220>
   <223> Primer
<400> 2
   ggcggggaat tcatgatgga cccaaactcg 30
<210> 3
   <211> 8
   <212> PRT
   <213> Epstein-Barr Virus
<220>
   <221 > MISC_FEATURE
   <223> Epitop within EBV protein BZLF1
<400> 3
   Arg Ala Lys Phe Lys Gln Leu Leu
   1 5

## Beschreibung

Die Erfindung betrifft ein Verfahren zum Einschleusen von Polypeptiden in Zellen. Die Erfindung betrifft ferner den Nachweis polypeptidspezifischer Immunzellen.

Der erworbene Ast des Immunsystems besteht aus einer humoralen (Immunglobuline) und einer zellulären Immunabwehr. Zelluläre und erregerspezifische Polypeptide werden von antigenpräsentierenden Zellen (APC) durch spezifische Spaltung aufgearbeitet und Bruchstücke (Epitope) davon zusammen mit MHC-Molekülen der Klassen-I und/oder -II auf der Zelloberfläche präsentiert. T-Zellen erkennen mittels ihres T-Zellrezeptors spezifisch Epitope, die im Komplex mit körpereigenen MHC-Proteinen präsentiert werden, und setzen eine Immunreaktion in Gang.

T-Zellen können anhand spezifischer Oberflächenproteine in verschiedene Effektorpopulationen untergliedert werden. CD4⁺CD8⁻ T-Helferzellen haben eine zentrale Bedeutung bei der Steuerung der Immunabwehr. Nach einer spezifischen Erkennung von Epitopen, die ihnen auf der Oberfläche von APC zusammen mit MHC-Proteinen präsentiert werden, regulieren sie durch die Sekretion unterschiedlicher Botenstoffe (beispielsweise Zytokine) die Produktion von Antikörpern durch B-Zellen (humoraler Ast der Immunantwort) und die Aktivierung CD4⁻CD8⁺ zytotoxischer T-Zellen (CTL) (zellulärer Ast der Immunantwort).

Die Bedeutung von CD4⁻CD8⁺ CTL liegt in der Erkennung und Zerstörung entarteter und von Mikroorganismen oder Parasiten befallener Zellen und Gewebe. T-Zellen stellen somit einen bedeutenden Schutzmechanismus des erworbenen Immunsystems zur Verhinderung und Kontrolle mikrobiell, insbesondere virusbedingter Erkrankungen und zur Erkennung und Zerstörung entarteter körpereigener Zellen dar. Neben diesen T-Zellpopulationen wurde von verschiedenen Arbeitsgruppen eine weitere Population zirkulierender T-Zellen beschrieben, die einen doppelt positiven CD4⁺CD8^{dim} Phänotyp aufweist. CD4⁺CD8^{dim} T-Zellen exprimieren CD8αα Homodimere und sind mit einer geringen Häufigkeit (weniger als 2% der Gesamtpopulation CD3⁺ T-Zellen) im Blut nachweisbar. Eine transiente oder persistierende Expansion von CD4⁺CDS^{dim} T-Zellen wurde sowohl in gesunden Personen als auch in Probanden mit verschiedenen Erkrankungen, einschließlich Infektionen mit unterschiedlichen Viren, beispielsweise dem humanen Immundefizienzvirus Typ-1 (HIV-1) und dem humanen Cytomegalievirus (HCMV), sowie Patienten mit verschiedenen Autoimmunerkrankungen beobachtet.

Desweiteren wurden weitere Populationen antigenspezifischer T-Zellen, die sogenannten CD56⁺CD8⁺ und CD56⁻CD57⁺CD8⁺ NKT-Zellen beschrieben. Diese Zellpopulationen exprimieren sowohl einen T-Zellrezeptor als auch klassische NK Zellmarker, und sind ebenfalls mit geringer Häufigkeit (2 bis 5% beziehungsweise 5 bis 10%) in peripheren blutmononukleären Zellen (PBMC) nachweisbar. Über die Bedeutung dieser Zellpopulationen bei der Kontrolle mikrobieller Infektionen und Tumore ist bislang nur wenig bekannt.

Professionelle APC, wie dendritische Zellen, Monozyten, Makrophagen, aber auch nicht professionelle APC wie B-Zellen spielen sowohl bei der Auslösung einer T-Zell-Antwort gegen körperfremde Immunogene als auch bei der Induktion einer T-Zell-Toleranz gegen körpereigene Gewebe eine zentrale Rolle. Die Aktivierung und Proliferation von T-Zellen erfolgt durch das gleichzeitige Auslösen von zwei Signalen. Das erste Signal wird durch den T-Zellrezeptor, der ein Epitop im Zusammenhang mit MHC auf der Oberfläche von APC erkennt, in die T-Zelle geleitet. Das zweite kostimulatorische Signal wird durch die spezifische Interaktion der kostimulatorischen Moleküle B7.1 (CD80) oder B7.2 (CD86) auf der APC mit dem zugehörigen Rezeptor (CD28) auf der Oberfläche der T-Zelle vermittelt. In Abwesenheit des kostimulatorischen Signals wird die epitopspezifische T-Zelle anerg. Anergie beschreibt einen Zustand, in dem die T-Zellen sich nicht vermehren und nicht auf ein Antigen reagieren kann.

Die Beschaffenheit eines Polypeptids bestimmt maßgeblich über die Effizienz und Route der Epitopprozessierung und Präsentation durch eine APC. Zudem wird der Aktivierungsgrad einer APC und somit das Profil der induzierten Immunantwort durch die Darreichungsform eines Polypeptides beeinträchtigt. So beeinflussen die Konzentration und biochemischen Eigenschaften eines Polypeptids, sowie die An- oder Abwesenheit von immunmodulatorischen Substanzen (insbesondere bakterielle Komponenten, wie Lipopolysaccharide (LPS), Nukleinsäuren (CpG-haltige DNS) und Polypeptide (z.B. Flagellin)) maßgeblich, ob der zelluläre (T Helfer-1 (Th-1)-Typ vermittelte Immunität) oder humorale Ast (T Helfer-2 (Th-2)-Typ-vermittelte Immunantwort) des Immunsystems aktiviert wird oder ob die Immunantwort tolerogen verläuft.

Bislang wurden nur sehr wenige Verfahren zum Einbringen von Polypeptiden in Säugerzellen beschrieben. So wurden bislang beispielsweise mechanische Verfahren der Mikroinjektion und Elektroporation mit wechselndem Erfolg zum Proteintransfer in Zellen verwendet (Mi *et al.* (2000); Mol. Ther. 2:339; Schwarze *et al.* (2000); Trends Cell Biol. 10:290). Weitere Verfahren zum Polypeptidtransfer in Zellen beruhen auf der Verwendung von Proteintransduktionsdomänen (PTD). Diese 10 bis 35 Aminosäuren umfassenden Aminosäuresequenzen entstammen beispielsweise dem HIV Tat Protein, dem Herpes Simplex Virus (HSV) VP22 Protein oder Antennapedia. Zudem wurden mittels Phagenbibliotheken synthetische PTD Sequenzen ermittelt. Die Membrangängigkeit von Polypeptiden kann durch deren Koppelung dieser mit PTD erheblich gesteigert werden. Weitere beschriebene Verfahren zum Proteintransfer in Zellen beruhen auf der Verwendung verschiedener kationischer Lipidformulierungen beziehungsweise dem Einbau von Polypeptiden in ISCOM^{®} Partikel (CSL Limited, Victoria, Australien). All diese Verfahren sind für einen routinemäßigen Gebrauch zu arbeits- oder kostenintensiv. Zudem besitzen viele der partikulären Transfersysteme zelltoxische (beispielsweise Liposomen) oder modulatorische Eigenschaften (ISCOM^{®} Partikel), welche die natürlichen Eigenschaften der behandelten Zellen nachhaltig beeinträchtigen können.

Unter Berücksichtigung der Bedeutung der zellulären Immunantwort für die Kontrolle mikrobieller Infekte und Tumore werden derzeit vielfältige neue Strategien zur *in vivo* Induktion der Epitöppräsentation auf MHC Klasse-I und -II Proteinen in Immunzellen getestet. Diese beinhalten den Einsatz von (Lipo-) Peptiden, (Lipo-) Proteinen, partikulären Immunogenen, lebend attenuierten Bakterien und Viren, rekombinanten Lebendimpfstoffen (auf der Basis verschiedener rekombinanter Bakterien und Viren) und DNS-Impfstoffen. Weiterhin stellen *ex vivo* behandelte autologe APC, welche spezifische Peptide im Kontext mit MHC Proteinen der Klassen-I und -II präsentieren, ein geeignetes Reagenz zur Induktion polypeptidspezifischer Immunantworten, insbesondere bei therapeutischen Behandlungen dar. Mit Tumorextrakten, beziehungsweise Zelllysaten gepulste APC haben sich in früheren Arbeiten zur gleichzeitigen Induktion von CD4⁺ und CD8⁺ T-Zellantworten als geeignet erwiesen (Herr *et al.* (2000), Blood, **96:**1857).

Derzeit sind verschiedene Verfahren zur Stimulation unterschiedlicher Populationen von Immunzellen verfügbar, welche in unterschiedlichem Ausmaß für den Nachweis spezifischer Populationen antigenspezifischer Immunzellen geeignet sind.

Die direkte Beladung membranständiger MHC-Proteine mit Peptiden definierter Länge (optimalerweise 8-11 Aminosäuren für die Beladung von MHC Klasse-I Proteinen, sowie optimalerweise 10 bis 20 Aminosäuren für die Beladung von MHC Klasse-II Proteinen) stellt eine vielfach verwendete Methode zur Stimulation definierter Populationen von Immunzellen, insbesondere CD8⁺ zytotoxischer Zellen (CTL) und CD4⁺ T-Helferzellen dar. Wesentliche Einschränkungen bei der Verwendung dieses Stimulationsverfahrens für die gleichzeitige Messung unterschiedlicher Populationen von Immunzellen, liegen jedoch darin, dass Peptide unterschiedlicher Größe spezifisch auf MHC Proteinen der Klassen-I oder -II präsentiert werden, wodurch bei Verwendung definierter Peptide keine gleichzeitige Bestimmung von CD4⁺ T-Helferzellen und CD8⁺ zytotoxischen T-Zellen möglich ist. Zudem unterliegt die spezifische Erkennung von T-Zellepitopen einer MHC Restriktion; das heißt Personen, die unterschiedliche MHC Proteine exprimieren, erkennen innerhalb eines Polypeptids unterschiedliche Epitope, wodurch die Analyse polypeptidspezifischer T-Zellen in Probanden mit variablem MHC-Muster erheblich erschwert wird. Somit können mit dieser Methode nur T-Zellen gezielt erfasst werden, welche gegen bekannte Epitope im Kontext mit definierten MHC Proteinen gerichtet sind.

Dagegen eignen sich rekombinant mittels unterschiedlicher Bakterien, sowie Insekten-, Hefe-, oder Säugerzellen hergestellte Polypeptide zum Nachweis Polypeptid-spezifischer CD4⁺ T-Helferzellen, unabhängig von der MHC Restriktion der Spenders und der detaillierten Kenntnis der in einem Polypeptid lokalisierten T-Zellepitope. Jedoch werden rekombinante Polypeptide fast ausschließlich über den MHC Klasse-II Prozessierungs- und Präsentationsweg in APC aufgenommen und aufgearbeitet, womit sich dieses Verfahren ausschließlich zum Nachweis CD4⁺ T-Helferzellen eignet.

Ferner wurden verschiedene Verfahren zur Denaturierung von Polypeptiden beschrieben, die es ermöglichen, diese Polypeptide dem MHC Klasse-I und MHC Klasse-II Prozessierungs- und Präsentationsweg zuzuführen. Diese Verfahren beinhalten beispielsweise eine Behandlung von 'Polypeptiden mit Hitze oder Natriumdodecylsulfat (SDS). Diese Verfahren erwiesen sich als geeignet, um eine Epitoppräsentation auf MHC Klasse-I und -II Molekülen in murinen APC zu erzielen (Schirmbeck *et al*. (1994), Eur. J. Immunol., 24:2068); (Schirmbeck *et al.* (1995), Vaccine, 13:857). In diesen Arbeiten wurde gezeigt, daß in unterschiedlicher Weise denaturierte Proteine mittels verschiedener Mechanismen in die APC aufgenommen werden und sich in ihrer Effizienz zur Induktion einer Peptidbeladung von MHC Klasse-I Polypeptiden unterscheiden. So induzierten mit dem Verfahren der Hitzeinaktivierung (1 Stunde bei 60° C oder 15 min bei 100°C) behandelte Polypeptide im Vergleich zu SDS behandelten Proteinen nur eine geringe Stimulation der Epitoppräsentation auf MHC Klasse I Proteinen in behandelten murinen APCs. Das Verfahren der SDS Denaturierung erwies sich dagegen für eine Verwendung im humanen Zellkulturen aufgrund der hohen Toxizität als wenig geeignet.

Ein weiteres Verfahren zur Stimulation der MHC Klasse-I und -II Präsentation von Epitopen auf APC beruht auf dem Einbau von Polypeptiden in partikuläre Strukturen, beispielsweise Liposonen, partikuläre Trägersubstanzen, virusähnliche Partikel oder Lipoproteinpartikeln. Die ersten Studien wurde Eignung von HIV-1 Pr55^{gag} Virus-ähnlichen Partikeln zur Diagnostik von CD4⁺ T-Helferzellen und CTL belegt (Sester *et al.* (2000), AIDS, **14**:2653-60). Die Herstellung partikelgebundener Polypeptide ist jedoch aufwendig und kostenintensiv. Ferner eignen sich diese Antigene nicht zur Diagnostik von anderen Immunzellpopulationen, beispielsweise CD4⁺CD8^{dim} zytotoxischen T-Zellen, CD56⁺CD8⁺- und CD56⁻CD57⁺CD8⁺ NKT-Zellen.

Ein weiteres Verfahren zur Stimulation der MHC Klasse-I und -II Präsentation von Epitopen auf APC beruht auf dem Einbringen von für die gewünschten Polypeptide kodierenden Polynukleotiden mittels Plasmiden, nichtviralen oder viralen Vektoren. Ein Nachteil bei der Verwendung von Plasmiden für diagnostische Zwecke besteht in der geringen Effizienz des Nukleinsäuretransfers in APC mittels der bislang verfügbaren Transfektionsverfahren, beispielsweise der Elektroporation oder Lipofektion. Virale oder bakteriellen Vektoren weisen oftmals im Vergleich zu Plasmiden deutlich gesteigerte Transfektionsraten von APCs auf. Diese Gentransfersysteme sind jedoch oftmals nicht immunologisch inert und modulieren die Fähigkeit von APC zur Epitopprozessierung und -präsentation von Polypeptiden (Jenne *et al.* (2001), *Trends Immunol*., **22**:102-7). Der Einsatz dieser auf Nukleinsäuren basierenden Verfahren ist zudem durch die aufwendige und kostenintensive Herstellung der Genfähren limitiert.

Desweiteren liegen bislang keine Anwendungsbeispiele bezüglich der Eignung dieser Systeme 'zur Diagnostik von anderen Immunzellpopulationen, beispielsweise CD4⁺CD8^{dim} zytotoxischen T-Zellen, CD56⁺CD8⁺ NKT und CD56⁻CD57⁺CD8⁺ NKT-Zellen vor.

Der nach erfolgter Stimulation unterschiedlicher Populationen von Immunzellen verwendete Nachweis der einzelnen Populationen sei hier kurz beschreiben. Der Nachweis von CD4⁺ T-Helferzellen erfolgt bislang durch die Bestimmung der Zellproliferation oder der von T-Zellen nach einer spezifischen Stimulation produzierten Botenstoffe (Zytokine). Der Nachweis der Zellproliferation erfolgt üblicherweise mittels eines Proliferationsassays durch die Bestimmung des in die DNS von proliferierenden Zellen eingebauten radioaktiven Isotops ³H Tritium. Die Bestimmung der Zytokinproduktion aus CD4⁺ T-Zellen nach einer polypeptidspezifischen Stimulation kann mittels eines Zytokin-ELISAs, eines ELISPOT Assays oder mittels der FACS-Technologie durch Bestimmung intrazellulärer Zytokine oder sezernierter Zytokine (FACS Sekretion-Assay) erfolgen.

Der Nachweis polypeptidspezifischer CD4⁻CD8⁺ zytotoxischer T-Zellen (CTL) erfolgt bislang durch den Nachweis ihrer spezifischen zytotoxischen Aktivität oder der von CTL nach einer spezifischen Stimulation produzierten Botenstoffe (Zytokine), insbesondere von Interferon-γ (IFN-γ). Der Nachweis der Zytotoxizität erfolgt üblicherweise mittels eines klassischen Chromfreisetzungstests oder adäquater nicht radioaktiver Verfahren, bei denen die Freisetzung von Enzymen oder ATP aus Zielzellen infolge einer spezifischen Lyse durch die Effektorzelle mit zytotoxischen Eigenschaften gemessen wird. Die Bestimmung der Zytokinproduktion aus CD8⁺ T-Zellen nach einer epitopspezifischen Stimulation kann mittels eines Zytokin-ELISAs, eines ELISPOT Assays oder mittels der FACS-Technologie durch Bestimmung intrazellulärer Zytokine, oder sezernierter Zytokine (FACS Sekretion-Assay) erfolgen. Als Markerzytokin für die Anwesenheit von CTL dient üblicherweise IFN-γ. Zur Stimulation Epitop- beziehungsweise polypeptidspezifischer CTL werden bislang beispielsweise autologe APC verwendet, welche CTL Epitope in Verbindung mit MHC Proteinen der Klasse-I auf ihrer Oberfläche präsentieren. Die Induktion einer MHC Klasse-I vermittelten Epitop-Präsentation auf APC wird bislang durch Inkubation dieser mit epitoptragenden Peptiden geeigneter Länge (8 bis 11 Aminosäuren), durch Inkubation mit Lipopolypeptiden, partikulären oder in partikuläre Strukturen verpackten Polypeptiden, Lysaten polypeptidproduzierender Zellen, apoptotischen Zellen, sowie vitalen aber auch abgetöteten polypeptidproduzierenden Mikroorganismen, insbesondere rekombinanten Viren, Bakterien oder Hefen vermittelt.

Der Nachweis polypeptidspezifischer CD4⁺CD8^{dim} zytotoxischer T-Zellen erfolgt bislang durch die Bestimmung der IFN-γ Produktion nach einer spezifischen Stimulation von Zellen in Vollblut mittels inaktivierter Viruspartikel, beispielsweise gp120-depletiertem HIV-1 Antigen (Reimmune^{™} in inkomplettem Freund's Adjuvans) oder gereinigtem Cytomegalievirus (CMV) Lysat (Advanced Biotechnologies, Columbia, MD) (Suni *et al.* (2001), Eur. J. Immunol., 31:2512-20).

CD56⁺CD8⁺ und CD56⁻CD57⁺CD8⁺ NKT-Zellen besitzen ein sehr begrenztes T-Zellrezeptorrepertoire, was vermuten läßt, dass diese Zellpopulation nur eine begrenzte Zahl von MHC präsentierten Antigenen erkennen kann. Bislang wurden im humanen System noch keine Verfahren zum Nachweis von NKT-Zellen beschrieben.

Die Di- und Tetramertechnologie sind Verfahren zum Nachweis Epitop-spezifischer CD8⁺ zytotoxischer T-Zellen und CD4⁺ T-Helferzellen. Beschränkungen dieser Verfahren für einen breiten Einsatz in der T-Zelldiagnostik sind jedoch in den sehr hohen Kosten für die Herstellung der Di- und Tetramere begründet. Zudem ist die Di- und Tetramertechnologie bislang nur für eine begrenztes Repertoire von MHC Typen, insbesondere für häufige MHC Klasse-I Proteine, beispielsweise HLA A2 verfügbar. Zudem erlaubt diese Technik nur den Nachweis definierter Epitop-spezifischer T-Zellen. Die Bestimmung von T-Zellreaktivitäten gegen multiple Epitope ist mit diesem Verfahren nur mit einem großen Zeit- und Kostenaufwand möglich.

Die Stimulation von peripheren Blutzellen mit inaktivierten Viruspartikeln ist bislang das einzige beschriebene Verfahren zur Stimulation und Detektion von CD4⁺CD8^{dim} zytotoxischen T-Zellen. Diese Stimulanzien beinhalten jedoch ein komplexes Gemisch unterschiedlicher viraler und nichtviraler Polypeptide, sowie anderer zum Teil immunstimulatorischer Viruskomponenten, wie Nukleinsäuren, Lipide und Zucker. Eine genaue Zuordung der bestimmten T-Zellreaktivität zu definierten viralen Polypeptiden ist somit mit dieser Methode nicht möglich.

Daher liegt der vorliegenden Erfindung die Aufgabe zu Grunde, ein Nachweissystem bereitzustellen, mit dem man gleichzeitig polypeptidspezifische bzw. epitopspezifische T-Zell- und andere Immunzell-Populationen nachweisen kann. Ferner liegt der vorliegenden Erfindung die Aufgabe zu Grunde, ein neues Verfahren zum Einschleusen von Polypeptiden in Zellen, insbesondere in APCs, bereitzustellen. Der vorliegenden Erfindung liegt ferner die Aufgabe zu Grunde, ein neues Verfahren zur Induktion der MHC Klasse-I und -II Präsentation polypeptidspezifischer Epitope mittels APCs bereitzustellen.

Die Aufgabe wird durch den in den Patentansprüchen definierten Gegenstand gelöst.

Die folgenden Figuren dienen der Erläuterung der Erfindung.

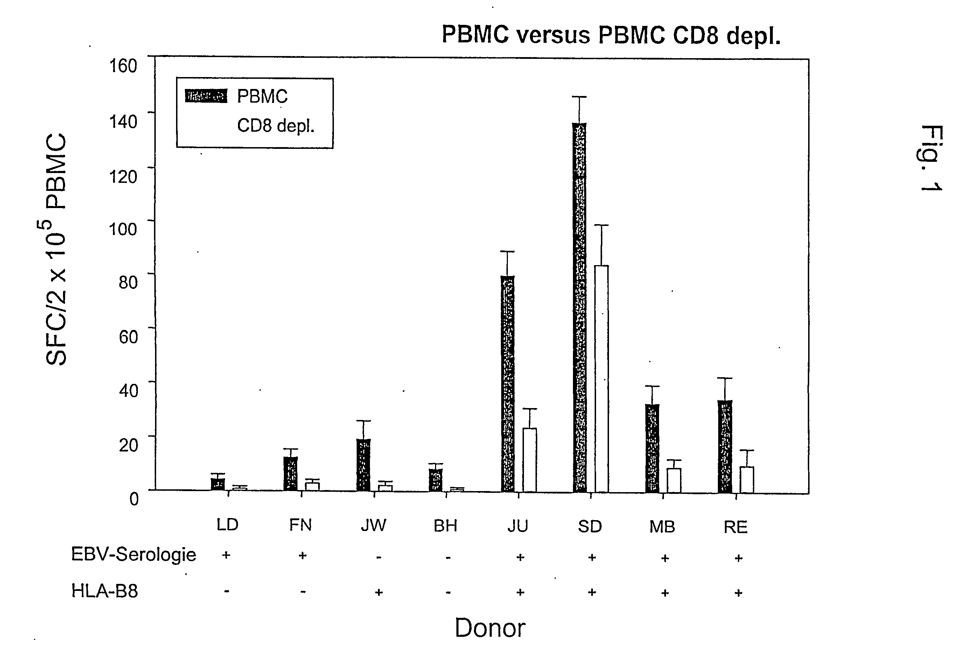

**Figur 1** zeigt in einer graphischen Darstellung die Anzahl IFN-γ sezernierender T-Zellen nach Inkubation von peripheren blutmononukleären Zellen (PBMC) mit Harnstoff-adjuviertem BZLF1 Polypeptid. Je 2 x 10⁵ PBMC beziehungsweise CD8⁺ T-zelldepletierte PBMC von je 2 HLA B8-negativen, EBV-positiven Spendern (LD, FN), einem HLA B8-negativen, EBV-negativen Spender (BH), einem HLA B8 positiven, EBV negativen Spender (JW), sowie 4 HLA B8-positiven, EBV-positiven Spendern (JU, SD, MB, RE) wurden in T-Zellmedium in ELISPOT Platten ausgesät und mit je 5µg/ml Harnstoff-adjuviertem BZLF1 Polypeptid für 24 Stunden inkubiert. Danach wurde die Zahl IFN-γ sezernierender Zellen mittels ELISPOT bestimmt. Die gezeigten Werte sind Mittelwerte ± Standardabweichung (SD) aus 6 unabhängigen Experimenten.

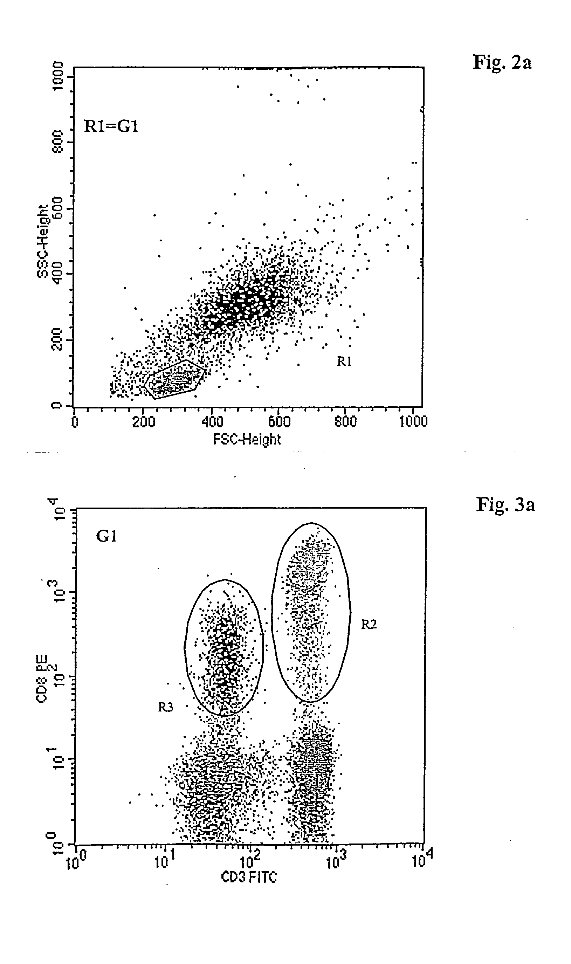

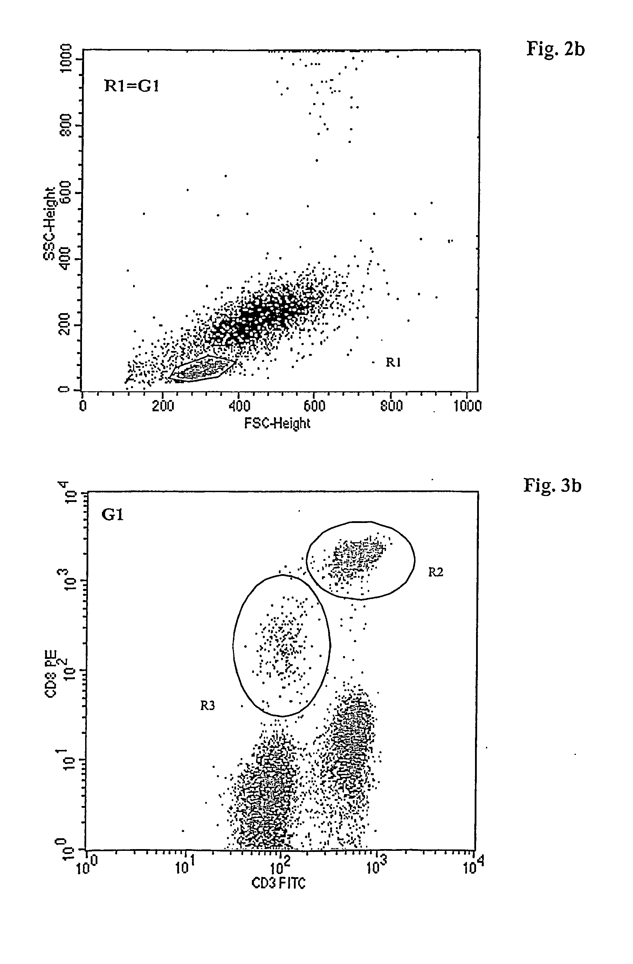

**Figur 2a und b** zeigen in der Dotplot Darstellung das Ergebnis einer durchflußzytometrische Analyse von Vollblut. Gezeigt ist ein FSC/SSC (Forward Scatter / Side Scatter) Dotplot, wobei der mit R1 (Region1) angegebene Messbereich der Lymphozytenpopulation entspricht. In den folgenden Figuren sind nur noch die in G1 enthaltenen Lymphozyten dargestellt. G1 entspricht der Population, die bei der Beschränkung der Messung auf ein Fenster gemäß dem Bereich R1 in Figur 2 bestimmt wurde.

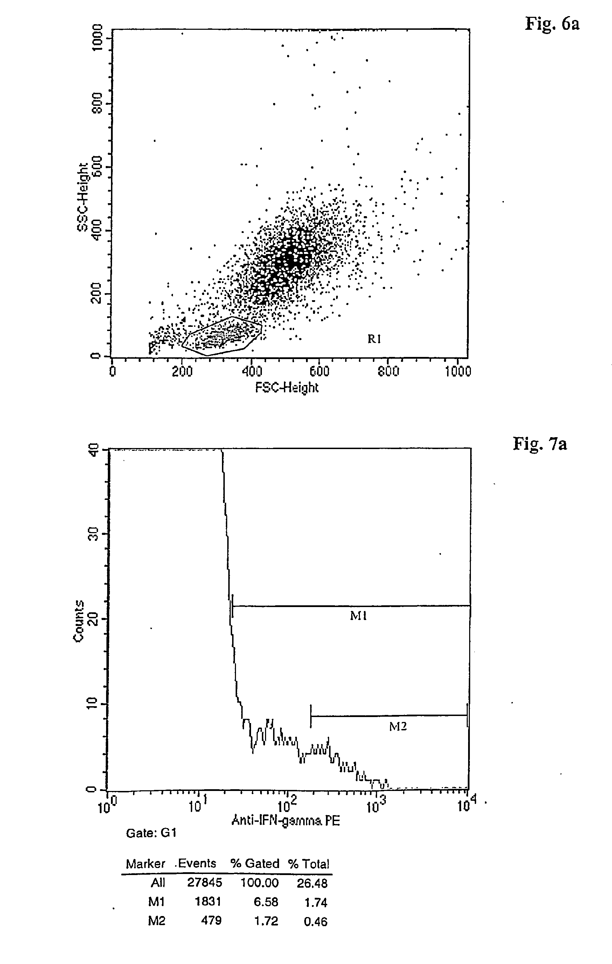

**Figur 3a und b** zeigen in der Dotplot Darstellung die Häufigkeit und Verteilung verschiedener Populationen CD3 und CD8 positiver Lymphozyten im Gesamtblut nach Stimulation mit Harnstoff-adjuviertem BZLF1 Polypeptid (Figur 3a) oder einem synthetischen BZLF1 Peptid (RAKFKQLL; Bogedain et al. (1995); J.Virol. 69:4872) (Figur 3B), welches ein bekanntes CTL Epitop beinhaltet. Gesamtblut eines HLA B8-positiven Epstein Barr Virus (EBV)-positiven Spenders wurde entweder mit Harnstoff-adjuviertem BZLF1 Protein oder mit dem synthetischen BZLF1 Peptid behandelt und für 6 Stunden stimuliert. Danach wurden die aktivierten Zellen mit anti-CD3-FITC und anti-CD8-APC gefärbt. Mit diesem Verfahren sind zwei distinkte Populationen CD3⁺CD8⁺ Zellen zu detektieren. Bei der Population, die eine starke Expression der CD3 und CD8 Polypeptide aufweist, (dargestellt in der Region 2 (R2)), handelt sich um klassische CD8⁺ zytotoxische T-Lymphozyten. Bei der in der Region 3 (R3) dargestellten Zellpopulation, welche eine reduzierte Expression der CD3 und CD8 Polypeptide aufweist, handelt es sich, wie in Figur 5 bestätigt wird, um CD56⁺ NKT-Zellen. Hierbei unterscheiden sich die Harnstoff-adjuviertem BZLF1 Polypeptide (Figur 3a) und das synthetische BZLF1 Peptid (Figur 3b) deutlich in ihrer Fähigkeit zur spezifischen Stimulation beider Populationen CD3⁺CD8⁺ Zellen.

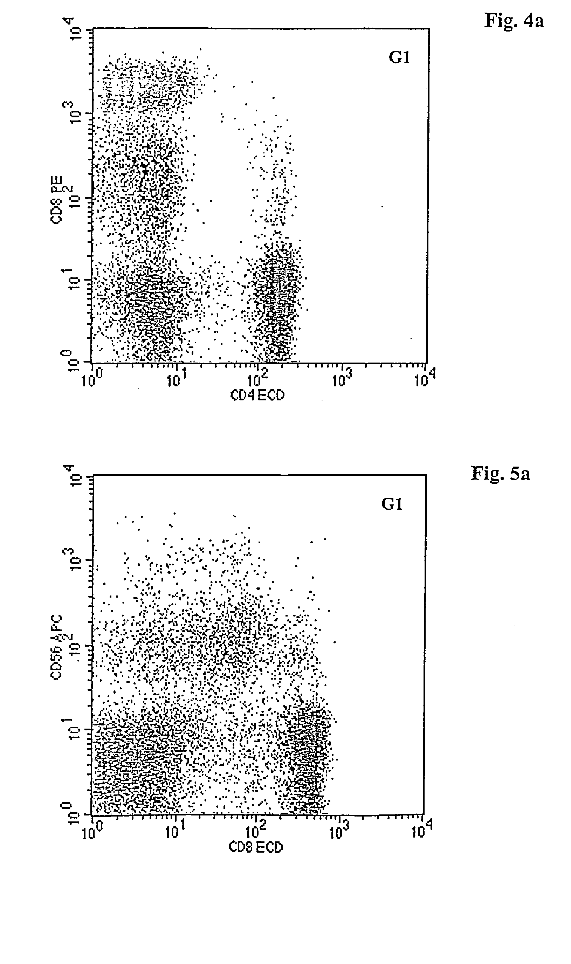

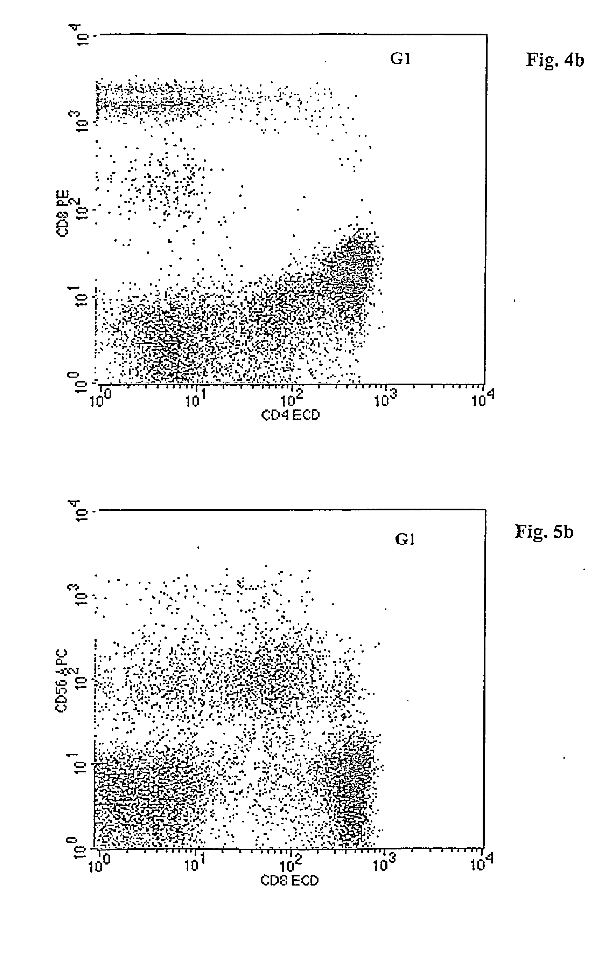

**Figur 4a und b** zeigen in der Dotplot Darstellung die Häufigkeit und Verteilung verschiedener Populationen CD4⁺ und CD8⁺ Lymphozyten im Gesamtblut nach Stimulation mit Harnstoff-adjuviertem BZLF1 Polypeptid (Figur 4a) oder einem synthetischen BZLF1 Peptid (Figur 4B), welches ein bekanntes CTL Epitop beinhaltet. Gesamtblut eines EBV-positiven Spenders wurde entweder mit Harnstoff-adjuviertem BZLF1 Protein oder dem synthetischen BZLF1 Peptid behandelt und für 6 Stunden stimuliert. Danach wurden die aktivierten Zellen mit anti-CD4-ECD und anti-CD8-APC gefärbt. Mit diesem Verfahren kann eine Population CD4 und CD8 doppelt positiver CD4⁺CDS^{dim} T-Lymphozyten detektiert werden. Nach Restimulation des Vollbluts eines EBV-positiven Patienten mit Harnstoff-adjuviertem BZLF1 Polypeptid (Fig. 4a) ist eine deutlich gesteigerte Zahl dieser doppelt positiven CD4⁺CD8^{dim} Zellen nachweisbar als nach Stimulation desselben Vollblutes mit dem BZLF1 Peptid (Fig. 4b).

**Figur 5a und b** zeigen in der Dotplot Darstellung Zellpopulationen welche die Expression der Oberflächenmarker CD8 und CD56 aufweisen. In beiden Figuren (Figur 5a und 5b) ist deutlich zu erkennen, dass die in Figur 2 mit R3 gekennzeichnete Population CD8-positiver Zellen, hier in blau dargestellt, CD56 auf der Zelloberfläche exprimiert. Somit stellen die in der Region R3 gezeigten Zellen die Population der NKT Zellen dar.

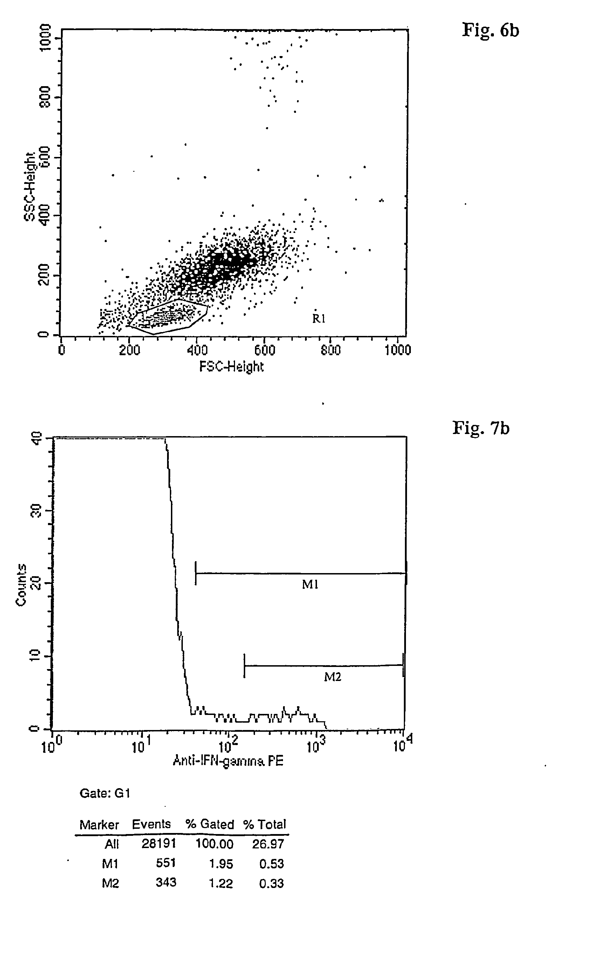

**Figur 6a und b** zeigen in der Dotplot Darstellung das Ergebnis einer durchflußzytometrischen Analyse von Zellen aus Vollblut nach Stimulation mit Harnstoff-adjuviertem BZLF1 Polypeptid (Figur 6a) beziehungsweise einem synthetischen BZLF1 Peptid (Figur 6B). Es handelt sich dabei um einen FSC/SSC (Forward Scatter / Side Scatter) Dotplot, wobei der mit R1 (Region1) angegebene Messbereich die Lymphozytenpopulation darstellt.

**Figur 7a und b** zeigen in der Histogramm Plot Darstellung das Ergebnis der IFN-γ Expression aus verschiedenen Populationen der gesamten in R1 erfassten Lymphozyten nach Stimulation mit Harnstoff-adjuviertem BZLF1 Polypeptid (Figur 7a) oder dem BZLF1 Peptid (Figur 7B). M1 und M2 sind hier als Marker gesetzt. M1 bezeichnet den Bereich, in dem die IFN-γ Expression als positiv zu werten ist. Alle Zellen, die links von dem gekennzeichneten Bereich M1 lokalisiert sind, wurden durch eine unspezifische Bindung des anti-IFN-γ Antikörpers innerhalb der Zellen unspezifisch angefärbt. Der durch M2 gekennzeichnete Bereich zeigt die IFN-γ Expression durch die reine Population CD3⁺CD8⁺ zytotoxischer T-Zellen. Die Differenz der in M1 und M2 dargestellten IFN-γ Werte kennzeichnet die IFN-γ Produktion durch schwach CD8⁺ Zellen mit NK-Zelleigenschaften. Nach Restimulation mit Harnstoff-adjuviertem BZLF1 Polypeptid (Figur 7a) kann im Vergleich zur Peptid-Stimulation eine deutlich erhöhte IFN-γ Produktion aus Zellen des Vollblutes sowie eine gesteigerte Stimulation der Population schwach CD8⁺ Zellen mit NK-Zelleigenschaften beobachtet werden.

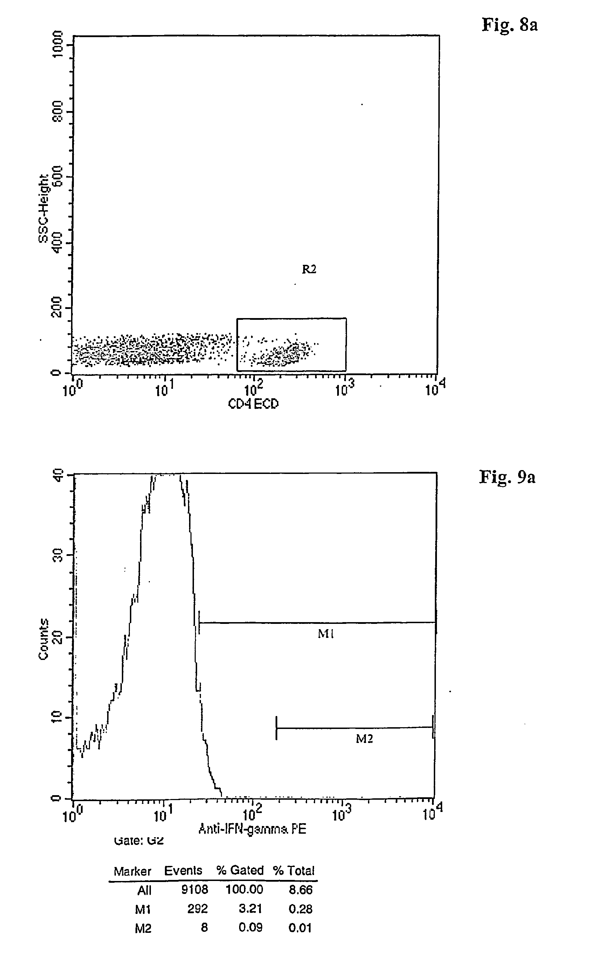

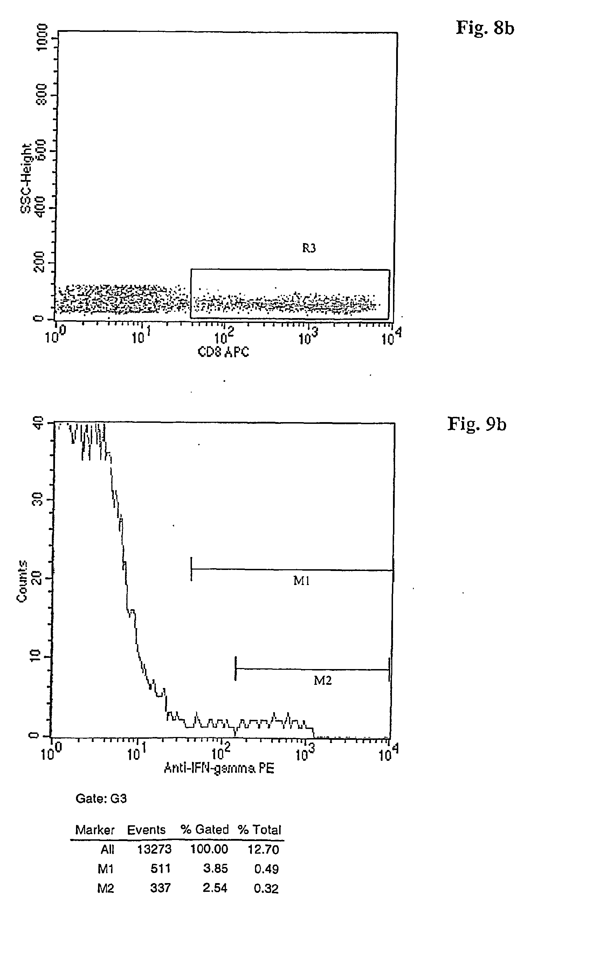

**Figur 8a und b** zeigen in der Dotplot Darstellung das Ergebnis der Expression des Oberflächenmarkers CD8 gegen den Side Scatter (Granularität der Zellen) von Zellen aus Vollblut nach Stimulation mit Harnstoff-adjuviertem BZLF1 Polypeptid (Figur 8a) oder dem synthetischen BZLF1 Peptid (Figur 8b). Mit dieser Einstellung können CD8⁺ zytotoxische T-Lymphozyten dargestellt werden. Diese Zellen sind hier als R3 Population benannt.

**Figur 9a und b** zeigen in der Histogramm Plot Darstellung das Ergebnis der IFN-γ Expression der in R3 dargestellten Population CD8⁺ zytotoxischer T-Zellen aus Vollblut nach Stimulation mit Harnstoff-adjuviertem BZLF1 Polypeptid (Figur 9a) oder dem synthetischen BZLF1 Peptid (Figur 9b). Die Marker M1 und M2 sind entsprechend der Figur 7a und b gesetzt. Diese Abbildung zeigt, dass Harnstoff-adjuviertem BZLF1 Protein und das synthetische BZLF1 Peptid gleichermaßen zur Bestimmung BZLF1-spezifischer zytotoxischer T-Zellen geeignet sind. Das gleichzeitige Auslesen von CD4⁺CD8^{dim} und CD8⁺ T-Zellen war jedoch nur nach Inkubation der Zellen mit Harnstoff-adjuviertem BZLF1 Polypeptid möglich.

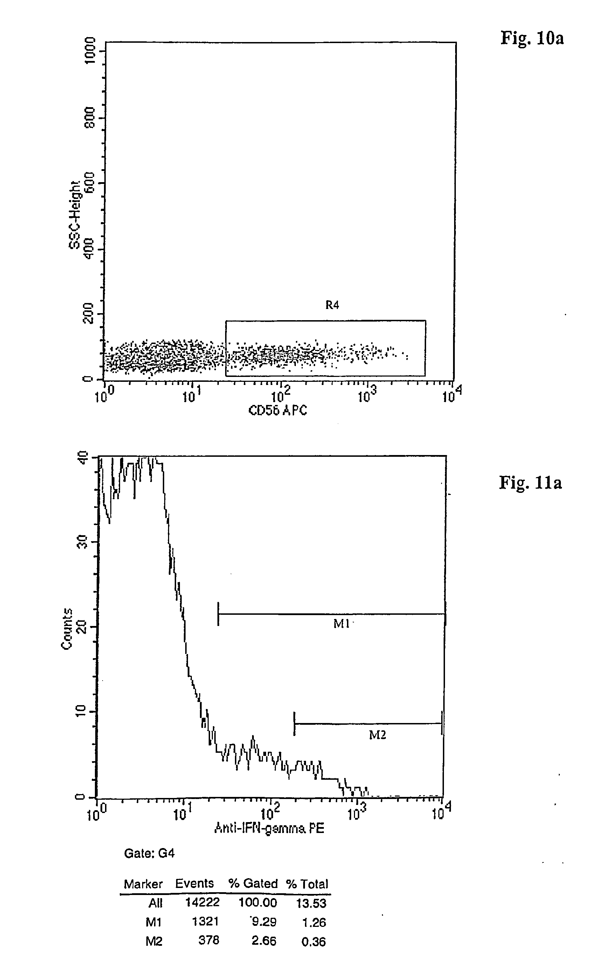

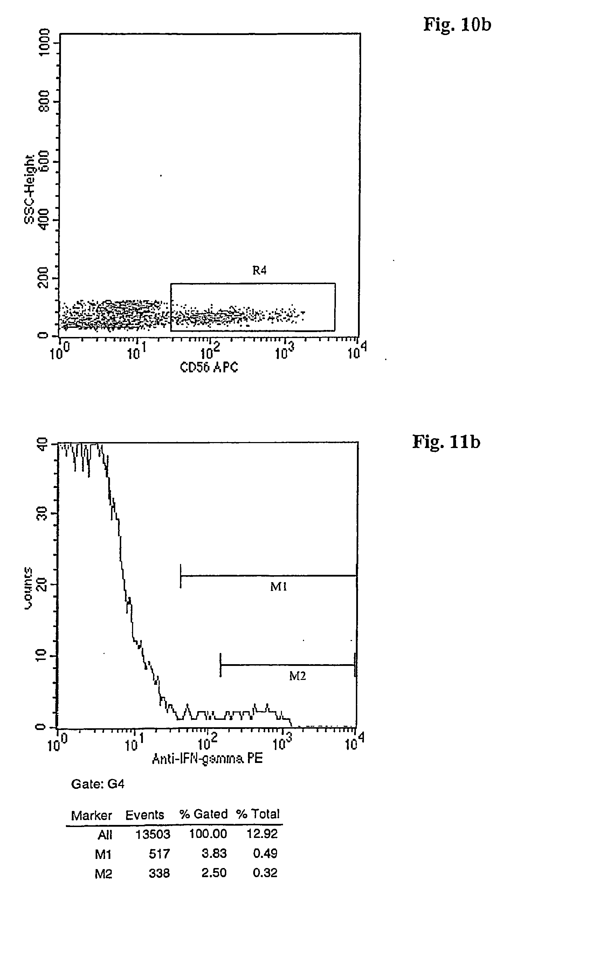

**Figur 10a und b** zeigen in der Dotplot Darstellung das Ergebnis der Expression des Oberflächenmarkers CD56 gegen den Side Scatter von Zellen aus Vollblut nach Stimulation mit Harnstoff-adjuviertem BZLF1 Polypeptid (Figur 10a) oder dem synthetischen BZLF1 Peptid (Figur 10b). Mit diesem Verfahren können CD56⁺ NKT-Zellen dargestellt werden. Diese Population ist hier als R4 benannt.

**Figur 11a und b** zeigen in der Histogramm Plot Darstellung das Ergebnis der IFN-γ Expression der mit R4 benannten NKT-Zellpopulation nach Stimulation mit Harnstoff-adjuviertem BZLF1 Polypeptid (Figur 11a) oder dem synthetischen BZLF1 Peptid (Figur 11b). Die Marker M1 und M2 sind entsprechend der Figur 7a und b gesetzt. Diese Abbildung belegt die effiziente Stimulation der schwach CD8-positiven NKT-Zellpopulation mittels Harnstoff-adjuviertem BZLF1 Polypeptid.

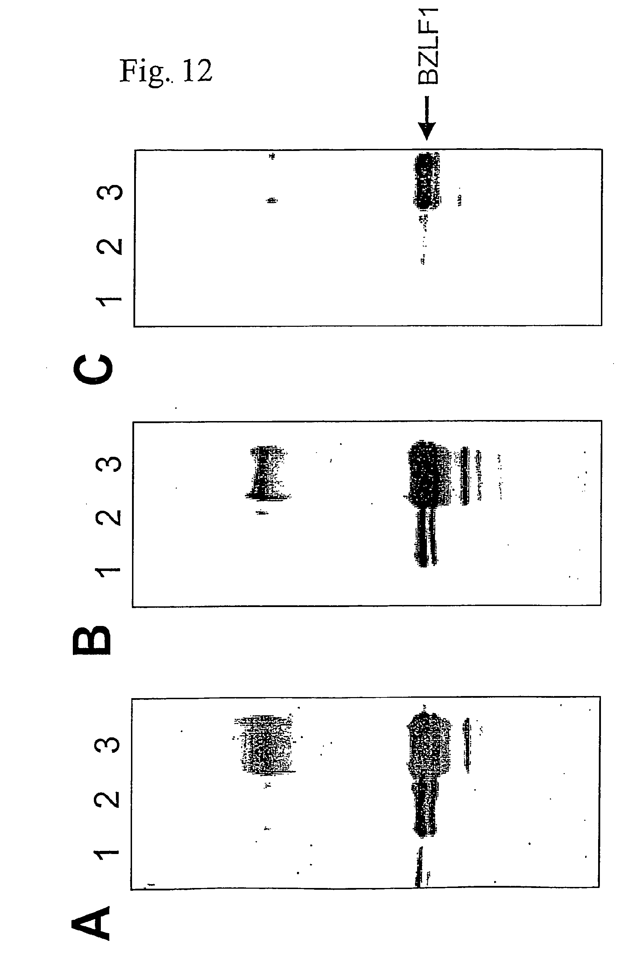

**Figur 12** zeigt im Immunoblot das Ergebnis der Reaktivität eines polyklonalen Kaninchenserum gegen rekombinantes BZLF1 Protein. Ein Kaninchen wurde intramuskulär mit 30 µg Harnstoff- und Hunters Titermax adjuviertem BZLF1 Polypeptid geimpft und nach 4 und 8 Wochen mit dem selben Immunogen nachimmunisiert. Nach weiteren 3 Wochen wurde Serum des Tieres in einer Verdünnung von (A) 1:2000, (B) 1:10000 und (C) 1:50000 im Immunoblot bezüglich ihrer Reaktivität gegenüber rekombinantem BZLF1-Protein in Konzentrationen von (1) 20 ng, (2) 100 ng und (3) 500 ng getestet.

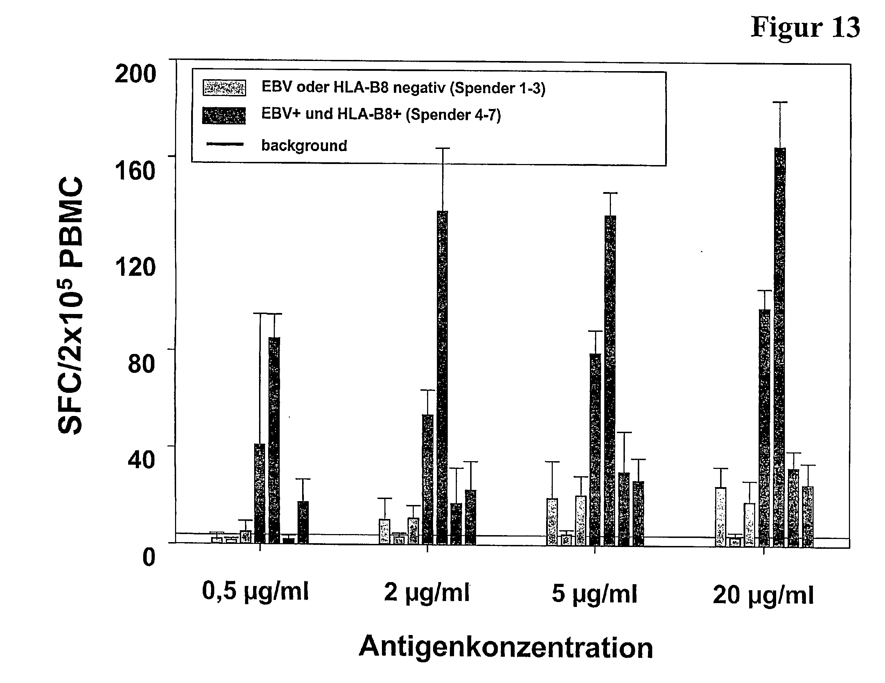

**Figur 13** zeigt schematisch die Dosisabhängigkeit der IFN-γ Sekretion nach Stimulation mit Harnstoff behandeltem BZLF-1 Protein. Die Untersuchungen zeigten, die BPMCs von 2 der 4 getesteten HLA-B8-positiven, EBV-positiven Spender bei allen getesteten BZLF-1 Konzentrationen eine im Vergleich zu den Negativkontrollen (Spender 1-3) deutlich gesteigerte Zahl IFN-γ produzierender BZLF-1-spezifischer T-Zellen aufwiesen. In diesen Experimenten waren bereits geringe Konzentrationen des Harnstoff-adjuvierten BZLF-1 Proteins ausreichend, um in 3 von 4 HLA-B8-positiven, EBV-positiven Spendern eine im Vergleich zu den Negativkontrollen (Spender 1-3) deutlich gesteigerte Zahl BZLF-1-spezifischer IFN-γ produzierender T-Zellen nachzuweisen.

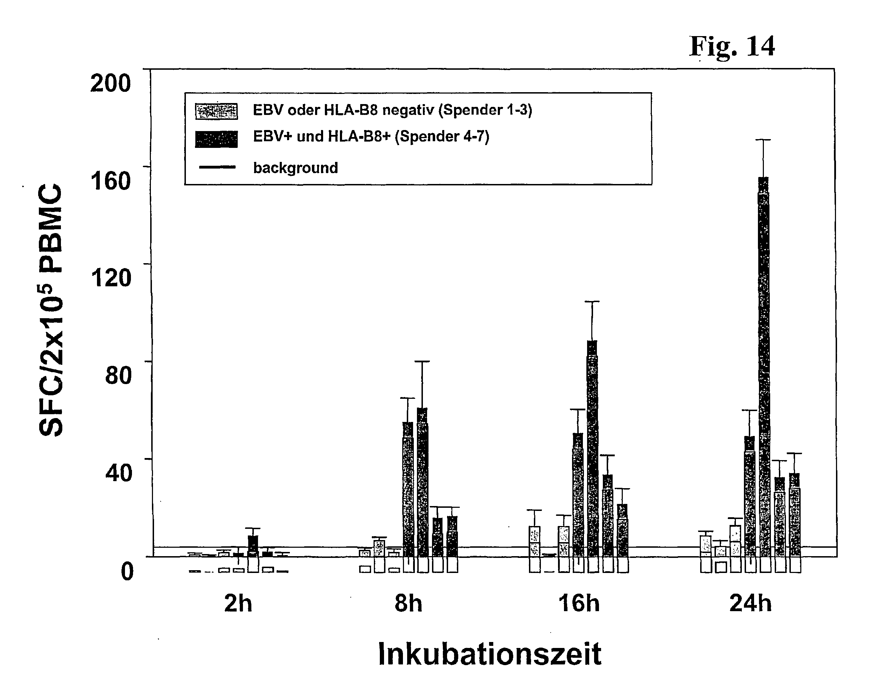

**Figur 14** zeigt schematisch den zeitlichen Verlauf der IFN-γ Sekretion nach Stimulation mit Harnstoff behandeltem BZLF-1 Protein. Die Untersuchungen zeigten, das bereits nach 8 Stunden Inkubation alle HLA-B8-positiven, EBV-positiven Spender (Spender 4-7) eine im Vergleich zu allen "Kontrollprobanden" (Spender 1-3) signifikant gesteigerte Zahl IFN-γ produzierender BZLF-1-spezifischer T-Zellen aufwiesen. Die maximale Zahl IFN-γ produzierender Zellen war spenderabhängig nach 16 bis 24 Stunden beobachtbar.

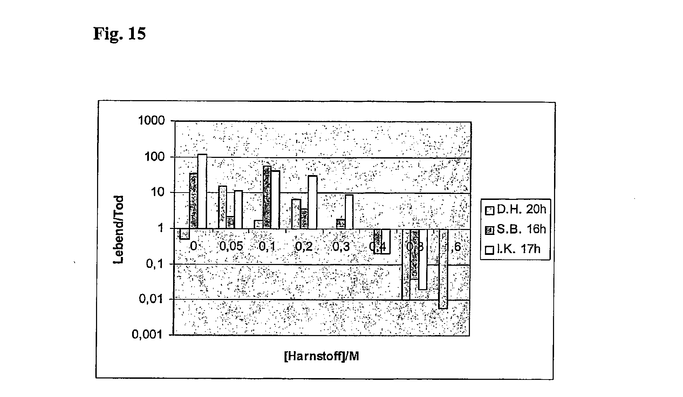

**Figur 15** zeigt in einer Graphik den Einfluss der Harnstoffkonzentration auf die Vitalität von PBMCs. D.H., S.B. und I.K. bezeichnen die Spender.

In der vorliegenden Erfindung werden allgemein gebräuchliche Abkürzungen für Nukleotide und Aminosäuren verwendet.

Der hier verwendete Begriff "genomisch" bezeichnet die Gesamtheit oder Fragmente des genetischen Materials eines Organismus.

Der hier verwendete Begriff "Polynukleotid" bezeichnet die polymere Form von Nukleotiden beliebiger Länge, vorzugsweise Desoxyribonukleotiden (DNS) oder Ribonukleotiden (RNS). Dieser Begriff bezeichnet nur die Primärstruktur des Moleküls. Der Begriff beinhaltet doppel- und einzelsträngige DNS und RNS z.B. Decoy- oder Antisense-Polynukleotide.

Der hier verwendete Begriff "Polypeptid" bezeichnet ein Polymer von Aminosäuren beliebiger Länge. Der Begriff Polypeptid umfasst auch die Begriffe Epitop, Peptid, Oligopeptid, Protein, Polyprotein und Aggregate von Polypeptiden. Ebenso sind in diesem Begriff Polypeptide eingeschlossen, die posttranslationelle Modifikationen z.B. Glykosylierungen, Acetylierungen, Phosphorylierungen und ähnliche Modifikationen aufweisen. Weiterhin umfasst dieser Begriff zum Beispiel Polypeptide, die ein oder mehrere Analoge von Aminosäuren (z.B. unnatürliche Aminosäuren), Polypeptide mit substituierten Verknüpfungen, sowie anderen Modifikationen, die Stand der Technik sind, aufweisen, unabhängig davon ob sie natürlicherweise vorkommen oder nicht natürlichen Ursprungs sind.

Der hier verwendete Begriff _{"}Harnstoff-adjuviert" bedeutet, dass ein Molekül z.B. ein Polypeptid in einer Lösung, vorzugsweise einer wässrigen Lösung, wie Wasser, einer Pufferlösung, einem Zellkulturmedium oder einer Körperflüssigkeit vorliegt, die eine bestimmte Harnstoffkonzentration aufweist. Der Begriff _{"}Harnstoff-adjuviert" bedeutet also, dass die Polypeptide nicht nur durch Harnstoff denaturiert werden sondern auch in einer Hamstoffhaltigen Lösung mit Zellen zur Transfektion in Kontakt gebracht werden. Die Harnstofflconzentration der Denaturierungs- und Transfektionslösung kann identisch oder verschieden sein. Ferner kann die Harnstofflösung NaCl und/oder DTE enthalten. Vorzugsweise weist die Harnstoffkonzentration der Transfektionslösung eine Endkonzentration im Bereich von etwa 0,001 bis etwa 0,8 mol/Liter, besonders bevorzugt im Bereich von etwa 0,001 bis etwa 0,2 mol/Liter, ferner von etwa 0,001 bis etwa 0,1 mol/Liter, insbesondere bevorzugt von etwa 0,001 bis etwa 0,01 mol/Liter, ferner besonders bevorzugt von etwa 0,01 bis etwa 0,2 mol/Liter, ferner von etwa 0,01 bis etwa 0,1 mol/Liter ferner besonders bevorzugt von etwa 0,1 bis etwa 0,8 mol/Liter aufweisen. Die Harnstoffkonzentration kann aber auch weniger als 0,001 mol/Liter, oder mehr als 0,8 mol/Liter betragen. Falls es auf eine hohe Gesamtzahl der lebenden Zellen und auf ein Verhältnis von lebenden zu toten Zellen ankommt, bei dem die lebenden Zellen überwiegen, sollte die Harnstoffkonzentration der Transfektionslösung weniger als 0,3 mol/Liter betragen, vorzugsweise z.B. 2,9, 2,8, 2,7, 2,6, 2,5 oder irgendeine Konzentration kleiner als 0,3 mol/Liter.

Die Begriffe "gereinigt", "aufgereinigt" und "isoliert" bedeuten, daß ein Molekül z.B. ein Polypeptid oder eine Nukleotidsequenz, in möglichst vollständiger Abwesenheit von biologischen Makromolekülen vergleichbarer Art vorliegt. Die Begriffe bedeuten ein anteiliges Gewicht des gewünschten Produktes von mindestens 75%, vorzugsweise von mindestens 85%, besonders bevorzugt von mindestens 95% und insbesondere bevorzugt von mindestens 98%, am Gesamtgewicht der vorliegenden biologischen Makromoleküle (Wasser, Puffer und andere kleine Moleküle, insbesondere Moleküle mit einer molekularen Masse von weniger als 1000 werden nicht zu den biologischen Makromolekülen gerechnet).

Der hier verwendete Begriff "Epitop" bezeichnet den Bereich eines Polypeptides, das antigene Eigenschaften besitzt und beispielsweise als Erkennungsstelle von T-Zellen oder Immunglobulinen dient. Im Sinne dieser Erfindung sind Epitope beispielsweise solche Bereiche von Polypeptiden, die von Immunzellen wie z.B. CD4⁺ T-Helferzellen, CD8⁺ zytotoxischen T-Zellen, CD4⁺CD8^{dim} zytotoxische T-Zellen, CD56⁺CD8⁺ sowie CD56⁻CD57⁺CD8⁺ NKT-Zellen oder CD4⁺CD25⁺ T-Suppressorzellen erkannt werden. Ein Epitop kann 3 oder mehr Aminosäuren umfassen. Üblicherweise besteht ein Epitop aus mindestens 5 bis 7 Aminosäuren oder, was häufiger ist, aus 8-11 Aminosäuren, oder aber aus mehr als 11 Aminosäuren, oder aber aus mehr als 20 Aminosäuren, seltener sogar aus mehr als 30 Aminosäuren. Der Begriff "Epitop" umfasst auch eine für das Epitop einzigartige räumliche Konformation. Diese räumliche Konformation ergibt sich aus der Abfolge der Aminosäuren im Bereich des Epitops.

Der hier verwendete Begriff "Mikroorganismus" bezeichnet Viren, prokaryotische und eukaryotische Mikroben wie z. B. Archebakterien, Bakterien, Einzeller und Pilze; die letztere Gruppe umfasst beispielsweise Hefen und filamentöse Pilze.

Der hier verwendete Begriff "Immunzellen" bezeichnet Lymphozyten mit regulatorischen oder zytolytischen Eigenschaften, wie beispielsweise CD4⁺ T-Helferzellen, CD8⁺ zytotoxische T-Zellen, CD4⁺CD8^{dim} zytotoxische T-Zellen, CD4⁺CD25⁺ T-Suppressorzellen, CD56⁺CD8⁺ und CD56⁻CD57⁺CD8⁺ NKT-Zellen, sowie NK Zellen.

Der hier verwendete Begriff "antigenpräsentierende Zelle" (APC) umfasst Zellen, die in der Lage sind Polypeptide aufzunehmen, zu prozessieren und Bruchstücke dieser Polypeptide (Epitope) dem Immunsystem in Verbindung mit MHC Klasse-I und MHC Klasse-II Proteinen zu präsentieren. Insbesondere umfasst der Begriff "antigenpräsentierende Zelle" dendritische Zellen (Langerhans Zellen), Monozyten, Makrophagen, B-Zellen aber auch vaskuläre endotheliale Zellen und verschiedene epitheliale, mesenchymale Zellen, sowie Mikrogliazellen des Gehirns.

Die vorliegende Erfindung betrifft ein Verfahren zum Polypeptidtransfer in Zellen, umfassend die folgenden Schritte:
a) Inkubieren von Polypeptiden mit einer Harnstofflösung,
b) Inkubieren.von Zellen mit den in der Harnstofflösung vorliegenden Polypeptiden.

Die Polypeptide können dabei synthetisch hergestellte Polypeptide sein oder mittels üblicher pro- oder eukaryontischer Expressionssysteme in verschiedenen Zellen exprimiert werden, wobei die nachfolgende Liste nur beispielhaft einige Zellen aufzählt, z.B. Bakterien wie *Bacillus subtilis, E. coli, Streptococcus cremoris* oder *Streptococcus lividans,* Hefezellen wie *Candida albicans, Candida maltosa, Hansenula polymorpha, Kluyveromyces fragilis, Saccharomyces cerevisiae, Schizosaccharomyces pombe,* oder *Yarrowia lipolytica,* Insektenzellen wie *Aedes aegypti, Autographa californica, Bombvx mori, Drosophila melanogaster, Spodoptera frugiperda,* oder *Trichoplusia ni,* Säugerzellen wie *Chinese hamster ovary* (CHO) Zellen, HeLa Zellen, *baby hamster kidney* (BHK) Zellen, Affennierenzellen (COS), oder Pflanzenzellen beruhen. Die genannten Systeme zur Expression von Polypeptiden sind vielfach publiziert und Stand der Technik.

Die verwendeten Polypeptide können mittels üblicher molekularbiologischer Verfahren, z.B. mittels Zellaufschluß, Nukleinsäureverdau, Verfahren zur Aufkonzentrierung von Proteinen, Affinitätschromatographie, Ionenaustauscherchromatographie und Gelfiltration, oder in Kombination mit Polypeptid-denaturierenden Verfahren, z.B. Säurefällung, Harnstoffbehandlung, Alkalibehandlung, Hitzebehandlung, Behandlung mit Natriumdodecylsulphat (SDS) oder Beschallung aufgereinigt sein. Diese Verfahren zur Aufreinigung von Polypeptiden können ferner in beliebiger Weise kombiniert werden.

Die Polypeptide können synthetische d.h. nicht natürlicherweise vorkommende Polypeptide sein oder in beliebigen Lebewesen z.B. in Säugetieren wie Menschen, Primaten, Maus, Ratte, Kaninchen, Schaf, Kuh, Schwein, oder in beliebigen Tieren, Parasiten, Mikroorganismen oder Viren vorkommen. Sie können aber auch Pflanzen und Algen entstammen. Sie können zudem Prionproteinen entstammen.

Die Konzentration der Harnstofflösung in Schritt a) liegt vorzugsweise im Bereich von etwa 0,01 mol/Liter bis etwa 10 mol/Liter oder auch darüber, bevorzugt bis zu etwa 0,1 mol/Liter, besonders bevorzugt im Bereich von etwa 0,1 bis etwa 0,5 mol/Liter, insbesondere bevorzugt im Bereich von etwa 0,5 bis etwa 5 mol/Liter, ferner insbesondere bevorzugt von etwa 5 bis etwa 10 mol/Liter, oder beträgt mehr als 10 mol/Liter.

Die Polypeptide können in jeder beliebigen Konzentrationen eingesetzt werden, vorzugsweise liegt die Konzentration im Bereich von etwa 0,01 bis etwa 50 µg/µl oder auch darüber, bevorzugt im Bereich von etwa 0,01 bis etwa 0,1 µg/µl, besonders bevorzugt von etwa 0,1 bis etwa 0,5 µg/µl, insbesondere bevorzugt von etwa 0,5 bis etwa 2 µg/µl, ferner insbesondere bevorzugt von etwa 2 bis etwa 10 µg/µl oder von etwa 10 bis etwa 50 µg/µl oder mit mehr als 50 µg/µl.

Die Inkubationszeit der Polypeptide mit der Harnstofflösung kann weniger als 10 Sekunden betragen, oder etwa 10 Sekunden bis einen Tag betragen, vorzugsweise etwa eine Minute, oder etwa eine Minute bis etwa eine Stunde, besonders bevorzugt etwa eine Stunde, oder auch mehr als einen Tag betragen. Die Polypeptide können weiterhin entweder gefroren oder bei einer Temperatur, bei der die Harnstofflösung sich in flüssigem Zustand befindet, für eine beliebig lange Zeit in der Harnstofflösung aufbewahrt werden, z.B. für mehr als eine Woche, für mehr als einen Monat oder auch für mehr als ein Jahr.

Die in der Harnstofflösung vorliegenden Polypeptide werden dann zur Inkubation der Zellen verwendet. Zu den Zellen wird ein so großes Volumen der Polypeptid/Harnstofflösung eingesetzt, dass die Konzentration der Polypeptide bei etwa 10⁶ Zellen im Bereich von etwa 0,1 bis etwa 200 µg oder auch höher liegt, vorzugsweise im Bereich von etwa 0,1 bis etwa 200 µg, besonders bevorzugt von etwa 0,1 bis etwa 2 µg, insbesondere bevorzugt von etwa 2 bis etwa 10 µg, ferner insbesondere bevorzugt von etwa 10 bis etwa 50 µg oder von etwa 50 bis etwa 200 µg Polypeptid.

Die Harnstoffkonzentration in Schritt b) sollte vorzugsweise eine Endkonzentration im Bereich von etwa 0,001 bis etwa 0,8 mol/Liter, besonders bevorzugt im Bereich von etwa 0,001 bis etwa 0,2 mol/Liter, ferner von etwa 0,001 bis etwa 0,1 mol/Liter, insbesondere bevorzugt von etwa 0,001 bis etwa 0,01 mol/Liter, ferner besonders bevorzugt von etwa 0,01 bis etwa 0,2 mol/Liter, ferner von etwa 0,01 bis etwa 0,1 mol/Liter ferner besonders bevorzugt von etwa 0,1 bis etwa 0,8 mol/Liter aufweisen. Die Harnstoffkonzentration kann aber auch weniger als 0,001 mol/Liter, oder mehr als 0,8 mol/Liter betragen. Falls es auf eine hohe Gesamtzahl der lebenden Zellen und auf ein Verhältnis von lebenden zu toten Zellen ankommt, bei dem die lebenden Zellen überwiegen, sollte die Harnstoffkonzentration in Schritt b) weniger als 0,3 mol/Liter betragen, vorzugsweise z.B. 2,9, 2,8, 2,7, 2,6, 2,5 oder irgendeine Konzentration kleiner als 0,3 mol/Liter. Zusätzlich kann die Harnstofflösung NaCl und/oder DTE enthalten, wobei die Konzentration von NaCl im Bereich von etwa 0,25 mmol/Liter bis etwa 0,2 mol/Liter und die von DTE im Bereich von etwa 0,25 nmol/Liter bis etwa 0,2 mmol/Liter liegt. Ferner kann die DTE-Konzentration kleiner 0,25 nmol/Liter oder größer 0,2 mmol/Liter sein.

Die Polypeptid/Harnstofflösung wird mit den Zellen für etwa 1 bis etwa 240 Stunden oder auch länger inkubiert, vorzugsweise für etwa 2 bis etwa 6 Stunden oder für etwa 6 bis etwa 12 Stunden oder für etwa 12 bis etwa 36 Stunden, oder für etwa 36 bis etwa 240 Stunden.

Das erfindungsgemäße Verfahren kann zum Einschleusen von Polypeptiden in beliebige Zellen also prokaryotische z.B. Bakterien und eukaryotische Zellen, z.B. Pilze wie Hefen und filamentöse Pilze, Insektenzellen, Vogel-, Reptilien-, Fisch-, Amphibien-, Säugerzellen, z.B. murine oder humane Zellen, z.B. antigenpräsentierende Zellen, verwendet werden.

Im Gegensatz zu den bislang beschriebenen Verfahren zum Polypeptidtransfer in Zellen zeichnet sich das erfindungsgemäße Verfahren insbesondere durch die universelle Anwendbarkeit, eine einfache Durchführbarkeit bei hoher Effizienz und einem sehr geringem Kostenaufwand aus.

Die mit dem erfindungsgemäßen Verfahren behandelten Zellen können im Bereich der Forschung, Diagnostik und Therapie und Prävention von Erkrankungen bei Tier und Mensch verwendet werden. So eignen sich die mit dem erfindungsgemäßen Verfahren erhaltenen APCs für prophylaktische und therapeutische Anwendungen zur Bekämpfung von Infektionserkrankungen und Tumoren. Ferner eignen sich die mit dem erfindungsgemäßen Verfahren erhaltenen APCs zur gleichzeitigen Diagnostik verschiedenster polypeptidspezifischer und immunogenstimulierbarer Immunzellen, umfassend CD4⁺CD8⁻ T-Helferzellen, CD4⁻CD8⁺ zytotoxische T-Zellen, CD4⁺CD8^{dim} zytotoxische T-Zellen, CD4⁺CD25⁺ T-Suppressorzellen, CD56⁺CD8⁺ und CD56⁻CD57⁺CD8⁺ NKT-Zellen, oder CD56⁺ NK Zellen.

Ein weiterer Aspekt der vorliegenden Erfindung betrifft die Verwendung der mit Harnstoff adjuviertem Polypeptide bei einer Vielzahl unterschiedlicher wissenschaftlicher, medizinischer und diagnostischer Anwendungen, z.B. zur Untersuchung der Bedeutung dieser Polypeptide bei zellulären Prozessen, zur Induktion humoraler und zellulärer Immunantworten im Versuchstier und im Menschen, für (a) die Gewinnung von Seren und Antikörpern für diagnostische, therapeutische und präventive Anwendungen, (b) zur Induktion geeigneter Immunantworten zum Schutz vor oder der Behandlung von mikrobiellen Infekten und Tumorerkrankungen, als (c) prophylaktische und therapeutische Vakzine oder (d) zur *ex vivo* Stimulation von APC für diagnostische, therapeutische und präventive Zwecke.

Daher betrifft die vorliegende Erfindung die Verwendung der mit Harnstoff adjuvierten Polypeptide zur Induktion spezifischer Antikörper und T-Zellen in Tieren, insbesondere in Säugern, z.B. in Mäusen, Ratten, Kaninchen, Schafen, Pferden, Rindern, Schweinen, Hunden, Katzen und Primaten. Die Harnstoff-adjuvierten Polypeptide können aber auch zur Induktion von humoralen und zellulären Immunantworten im Menschen verwendet werden. Hierbei können die Harnstoff-adjuvierten Polypeptide entweder alleine oder in Kombination mit immunstimulierenden Agenzien ("Adjuvanzien") verabreicht werden.

Besonders geeignete Adjuvanzien zur Steigerung der Effektivität der beschriebenen Impfstoffe/Impfstoffkombinationen beinhalten beispielsweise: (1) gelartige Adjuvanzien wie Aluminumsalze (Alum), wie Aluminumhydroxid, Aluminumphosphat, Aluminumsulfat und Calzium Phosphat; (2) mikrobielle Adjuvantien wie bakterielle Nukleinsäuren mit CpG Motiven, Endotoxine wie beispielsweise Monophosphoryl Lipid A, Exotoxine wie beispielsweise das Diphterie-, Cholera-, Tetanus-Toxoid, das hitze-labile Enterotoxin von *E. coli* und Muramyl Dipeptide wie beispielsweise MDP; (3) Öl Emulsionen und auf Emulsionen basierende Impfstoffe wie beispielweise inkomplettes Freunds Adjuvans (1FA), MF59, SAF und Ribi^{™} Adjuvanssystem (RAS), (Ribi Immunochem, Hamilton, Mont.); (4) partikuläre Adjuvanzien, wie beispielsweise immunstimulatorische Komplexe (ISCOMs), Liposomen, PLG Polymere, biologisch abbaubare Mikrosphären und Saponine (QS-21), und synthetische Adjuvanzien wie nichtionische Blockpolymere, Muramypeptid Analoge, Polyphosphazene und synthetische Polynukleotide und (5) Zytokine, wie beispielsweise Interleukine (IL-1, IL-2, IL-12 u.a.), Granulozyten/Makrophagen Kolonie-stimulierender Faktor (GM-CSF) oder Makrophagen Kolonie-stimulierender Faktor (M-CSF), sowie der Tumor Nekrose Faktor (TNF). Neben Adjuvantien können alle anderen Substanzen, die eine immunstimulierende Wirkung zur Steigerung der Effizienz der beschriebenen Impfstoffkompositionen aufweisen, eingesetzt werden. Eine Auflistung geeigneter, verfügbarer Adjuvantien wurde beispielsweise von F.R. Vogel zusammengestellt und ist über das *world wide web* unter folgender Adresse abrufbar (hppt://www.niaid.nih.gov/aidsvaccine/pdf/compendium.pdf).

Die beschriebenen Impfstoffkombinationen (z.B. die Harnstoff-adjuvierten Polypeptide in Verbindung mit einer pharmazeutisch akzeptierten Trägerkomponente und/oder einem Adjuvans) werden zu den Verdünnungslösungen, wie beispielsweise Wasser, Salzlösungen, Glyzerin, Ethanol zugegeben. Zudem können zusätzliche akzessorische Komponenten, wie anfeuchtende und emulgierende Agenzien, pH-puffernde Substanzen und ähnliche Komponenten in diesen Kompositionen vorhanden sein.

Üblicherweise werden diese Impfstoffkombinationen in injizierbarer Form, entweder als flüssige Lösungen oder Suspensionen vorliegen. Die immunogene Komposition kann ebenso in Liposomen emulgiert oder eingebaut werden, um im Sinne einer pharmazeutisch akzeptieren Trägerkomponente gesteigerte Adjuvanseigenschaften zu erzielen. Die Impfstoffkomposition kann in geeigneten Darreichungswegen verabreicht werden. Möglich sind dabei unter anderem orale, topische, intravenöse, intraperitoneale, intramuskuläre, intraarticuläre, subkutane, intranasale oder intradermale Darreichungswege. Die Impfstoffkomposition wird in der für die Indikation angemessenen Dosen verwendet. Die Bestimmung einer für unterschiedliche Organismen angemessenen Dosis ist Stand der Technik. Die beschriebenen Impfstoffkombinationen können entweder prophylaktisch oder therapeutisch eingesetzt werden. Desweiteren eignen sich mittels Harnstoff-adjuvierter Polypeptide modifizierte APC für therapeutische und präventive Anwendungen. Verfahren zur Gewinnung und *ex vivo* Expansion von APC, sowie zur Reinfusion *ex vivo* modifizierter APC in einen Organismus sind vielfach publiziert und Stand der Technik.

Ein weiterer Aspekt der vorliegenden Erfindung betrifft ein Verfahren zum Nachweis polypeptidspezifischer Immunzellen, umfassend die folgenden Schritte:
a) Inkubieren von Polypeptiden mit einer Harnstofflösung,
b) Inkubieren von APC-haltigen Zellkulturen oder Körperflüssigkeiten mit den in der Harnstofflösung vorliegenden Polypeptiden,
c) Inkubieren der nach Schritt b) erhaltenen APC-haltigen Zellkulturen oder Körperflüssigkeiten mit Immunzellen oder immunzellhaltigen Körperflüssigkeiten,
d) gleichzeitiger Nachweis und/oder Quantifizierung verschiedener Subtypen von Immunzellen, die spezifisch gegen die Polypeptide aus Schritt a) sind.

Schritt a) dieses erfindungsgemäßen Verfahrens ist identisch mit Schritt a) des Verfahrens zum Polypeptidtransfer in Zellen, so dass die entsprechenden vorstehenden Erläuterungen auch für das Verfahren zum Nachweis polypeptidspezifischer Immunzellen zutreffen.

Die in der Harnstofflösung vorliegenden Polypeptide werden dann zur Inkubation von APC-haltigen Zellkulturen oder Körperflüssigkeiten verwendet. Zu etwa 10⁶ Zellen wird ein so großes Volumen der Polypeptid/Harnstofflösung eingesetzt, dass die Konzentration der Polypeptide im Bereich von etwa 0,1 bis etwa 200 µg oder auch höher liegt, vorzugsweise im Bereich von etwa 0,1 bis etwa 200 µg, besonders bevorzugt von etwa 0,1 bis etwa 2 µg, insbesondere bevorzugt von etwa 2 bis etwa 10 µg, ferner insbesondere bevorzugt von etwa 10 bis etwa 50 µg oder von etwa 50 bis etwa 200 µg Polypeptid.

Die Harnstoffkonzentration in Schritt b) sollte vorzugsweise eine Endkonzentration im Bereich von etwa 0,001 bis etwa 0,8 mol/Liter, besonders bevorzugt im Bereich von etwa 0,001 bis etwa 0,2 mol/Liter, ferner von etwa 0,001 bis etwa 0,1 mol/Liter, insbesondere bevorzugt von etwa 0,001 bis etwa 0,01 mol/Liter, ferner besonders bevorzugt von etwa 0,01 bis etwa 0,2 mol/Liter, ferner von etwa 0,01 bis etwa 0,1 mol/Liter ferner besonders bevorzugt von etwa 0,1 bis etwa 0,8 mol/Liter aufweisen. Die Harnstoffkonzentration kann aber auch weniger als 0,001 mol/Liter, oder mehr als 0,8 mol/Liter betragen. Falls es auf eine hohe Gesamtzahl der lebenden Zellen und auf ein Verhältnis von lebenden zu toten Zellen ankommt, bei dem die lebenden Zellen überwiegen, sollte die Harnstoffkonzentration in Schritt b) weniger als 0,3 mol/Liter betragen, vorzugsweise z.B. 2,9, 2,8, 2,7, 2,6, 2,5 oder irgendeine Konzentration kleiner als 0,3 mol/Liter. Zusätzlich kann die Harnstofflösung NaCl und/oder DTE enthalten, wobei die Konzentration von NaCl im Bereich von etwa 0,25 mmol/Liter bis etwa 0,2 mol/Liter und die von DTE im Bereich von etwa 0,25 nmol/Liter bis etwa 0,2 mmol/Liter liegt. Ferner kann die DTE-Konzentration kleiner 0,25 nmol/Liter oder größer 0,2 mmol/Liter sein.

Die Polypeptid/Harnstofflösung wird mit den APC-haltigen Zellkulturen oder Körperflüssigkeiten für etwa 1 bis etwa 240 Stunden oder auch länger inkubiert, vorzugsweise für etwa 2 bis etwa 6 Stunden oder für etwa 6 bis etwa 12 Stunden oder für etwa 12 bis etwa 36 Stunden, oder für etwa 36 bis etwa 240 Stunden.

Die APC-haltigen Zellkulturen können gereinigte PBMC-Population (Leukapheresat), isolierte monozytäre Zellen oder eine separierte APC-Population, z.B. dendritische Zellen (Langerhans Zellen), Monozyten, Makrophagen oder B-Zellen sein. Der Begriff "APC-haltige Zellkulturen" wie hier verwendet bedeutet somit nicht nur in vitro in Kulturmedien gehaltene und vermehrte APC umfassende Zellen, sondern auch die einem Probanden, Patienten oder einem Tier entnommenen und aufgereinigten APC enthaltenen Zellpopulationen. Die APC-haltige Körperflüssigkeit ist vorzugsweise Vollblut oder Liquor.

Beispielsweise kann einem Probanden oder Patienten Blut oder eine andere APC-haltige Körperflüssigkeit entnommen werden. Die Körperflüssigkeit kann entweder direkt in Schritt b) des erfindungsgemäßen Verfahrens verwendet werden oder es können APC-haltige Zellpopulationen aufgereinigt und anschließend eingesetzt werden. Die Aufreinigung APChaltiger Zellpopulationen aus Blut oder anderen APC-haltigen Körperflüssigkeiten ist Stand der Technik und dem Fachmann bekannt.

Nach Inkubation der APC-haltigen Zellkulturen oder Körperflüssigkeiten mit den in der Harnstofflösung vorliegenden Polypeptiden werden die Zellen mit Immunzellen oder immunzellhaltigen Körperflüssigkeiten inkubiert. Die Immunzellen oder immunzellhaltigen Körperflüssigkeiten stammen vorzugsweise von demselben Probanden, Patienten oder Tier ab, von dem auch die APC-haltigen Zellkulturen oder Körperflüssigkeiten abstammen. Alternativ können die Immunzellen oder immunzellhaltigen Körperflüssigkeiten auch aus Probanden, Patienten oder Tieren mit einem zu den APC-haltigen Zellkulturen oder Körperflüssigkeiten kompatiblem MHC-Muster stammen.

Die Immunzellen können T-Zellen, z.B. CD4⁺ T-Zellen, CD8⁺ T-Zellen, CD4⁺CD8^{dim} T-Zellen, CD4⁺CD25⁺ Suppressor T-Zellen, aber auch andere Zellpopulationen wie beispielsweise CD56⁺CD8⁺ sowie CD56⁻CD57⁺CD8⁺ NKT-Zellen oder CD56⁺ NK-Zellen sein. Desweiteren umfasst der Begriff Immunzellen auch ein beliebiges Gemisch aus CD4⁺ T-Zellen, CD8⁺ T-Zellen CD4⁺CD8^{dim} T-Zellen, CD4⁺CD25⁺ T-Zellen, CD56⁺CD8⁺ sowie CD56⁻CD57⁺CD8⁺ NKT-Zellen und CD56⁺ NK-Zellen. Der Begriff "Immunzellen" wie hier verwendet bedeutet somit nicht nur *in vitro* in Kulturmedien gehaltene und vermehrte Immunzellen, sondern auch die einem Probanden, Patienten oder einem Tier entnommenen und aufgereinigten Immunzell-Populationen. Die immunzellhaltigen Körperflüssigkeiten sind vorzugsweise Vollblut oder Liquor. Verfahren zur Gewinnung und Reinigung definierter APC- und Immunzellpopulationen sind vielfach publiziert und Stand der Technik.

Ferner kann die in Schritt b) verwendete APC-haltige Zellkultur oder Körperflüssigkeit z.B. Vollblut, Liquor oder gereinigte PBMC bereits die nachzuweisenden Populationen von Immunzellen enthalten. In diesem Fall ist eine Zugabe von Immunzellen oder immunzellhaltigen Körperflüssigkeiten in Schritt c) nicht mehr erforderlich.

Die Inkubation in Schritt c) beträgt etwa 1 bis etwa 240 Stunden oder auch länger, vorzugsweise etwa 2 bis etwa 6 Stunden oder etwa 6 bis etwa 12 Stunden oder etwa 12 bis etwa 36 Stunden, oder etwa 36 bis etwa 240 Stunden unter geeigneten Kultivierungsbedingungen, beispielsweise bei 37° C in einer befeuchteten Atmosphäre mit 5 bis 8 % CO₂ in T-Zellmedium (RPMI 1640 mit 2 bis 10% Hitze inaktiviertem (30 min, 56°C) Humanserum oder fötalem Kälberserum (FKS), 2 mM Glutamin und 100 mg/ml Kanamycin oder Gentamycin (alle Komponenten von PanSystems, Aidenbach)). Andere geeignete Bedingungen (Variation der Medienkomposition, Temperatur, Luftfeuchtigkeit, Inkubationszeit) zur Kultivierung von APC und Immunzellen sind vielfach beschrieben und Stand der Technik.

Der Nachweis definierter Populationen polypeptidspezifischer Immunzellen beruht auf dem Befund, daß Immunzellen nach einer spezifischen Erkennung von Polypeptiden, welche gemeinsam mit MHC Proteinen der Klassen-II und/oder -I auf der Oberfläche von APC präsentiert werden, eine gesteigerte Expression charakteristischer Zytokine, insbesondere IFN-γ, bzw. IL4 und/oder IL5 aufweisen. Durch eine gemeinsame Analyse von Oberflächenproteinen, die für definierte Immunzellpopulationen charakteristisch sind und von Zytokinen können aus einem Gemisch unterschiedlicher Populationen von Immunzellen die Anwesenheit und/oder Konzentration definierter Populationen polypeptidspezifischer Immunzellen nachgewiesen werden. Der Nachweis und/oder die Quantifizierung in Schritt d) erfolgt also über den gleichzeitigen Nachweis von Oberflächenproteinen und Zytokinen.

Der Nachweis definierter Zellpopulationen über spezifische Oberflächenproteine erfolgt, beispielsweise über CD4 für T-Helferzellen, CD8 für zytotoxische T-Zellen, CD4 und CD8 für CD4⁺CD8^{dim} zytotoxische T-Zellen, CD56 für NK-Zellen, CD4 und CD25 für Suppressor T-Zellen, sowie CD56 und CD8 bzw. CD57 und CD8 für verschieden Populationen von NKT-Zellen. Spezifische Zustandsformen der Zellpopulationen (ruhende versus aktivierte Zellen versus Gedächtniszellen), sowie der Grad der Aktivierbarkeit können ferner durch den Nachweis zusätzlicher Oberflächenproteine (beispielsweise CD69, CD45RA, CCR7) und intrazellulärer Proteine (beispielsweise Granzym oder auch Perforin) bestimmt werden. Diese für definierte Zellpopulationen charakteristischen Oberflächenmarker sind vielfach publiziert und der Nachweis und die Charakterisierung verschiedener Populationen von Immunzellen mittels z.B. der FACS-Technologie sind Stand der Technik.

Der Nachweis der spezifischen Aktivierung von Immunzellen erfolgt nach Inkubation mit den nach Schritt b) erhaltenen APC-haltigen Zellkulturen oder Körperflüssigkeiten durch die Messung einer gesteigerten Zytokinproduktion der aktivierten Immunzellen. Hierbei produzieren z.B. CD4⁺ T-Helferzellen des T-Helfer 1 Typs (Th-1), CD8⁺ zytotoxische T-Zellen, CD4⁺CD8^{dim} zytotoxische T-Zellen, CD56⁺ NK-Zellen, sowie CD56⁺CD8⁺ bzw. CD57⁺CD8⁺ NKT Zellen nach spezifischer Stimulation verstärkt IFNγ, wohingegen CD4⁺ T-Helferzellen des T-Helfer 2 Typs (Th-2) eine gesteigerte Produktion der Zytokine IL4 und IL5 aufweisen. Die produzierten Zytokine können mittels vielfach publizierter Verfahren entweder intrazellulär oder nach Sekretion in den Überstand mittels zum Teil kommerziell erhältlicher Verfahren, beispielsweise mittels der FACS Technologie, einfach bestimmt werden. Der Nachweis ist auch über andere nach der spezifischen Aktivierung von Immunzellen produzierte Zytokine oder andere produzierte Marker möglich.

Die Bestimmung und Charakterisierung reaktiver Immunzellen kann z.B. mittels der *Fluorescence activated cell scan* (FACS) Technologie erfolgen. Dieses Verfahren erlaubt die Messung der Fluoreszenzintensität einzelner Zellen in einer gemischten Zellpopulation mit Hilfe eines Durchflußzytometers. Die durchflußzytometrische Analyse der Zellen erfolgt dann mittels eines FACS Gerätes, beispielsweise an einem FACS CALIBUR, Becton Dickinson (Franklin Lakes, NJ, USA), die Auswertung mit Hilfe des Programms Cell Quest (Becton Dickinson, Heidelberg).

Zum Nachweis der zuvor beschriebenen charakteristischen Oberflächenproteine und Zytokine mittels der FACS-Technologie sind Fluoreszenz-gekoppelte z.B. mit R-Phycoerythrin (R-PE), Peridin-Chlorophyll c (PerCP), Fluoescein (FITC), Texas Red (TX), Allophycocyanin (APC), Tandem PE-TX, Tandem PE-Cy5, PE-Cy7 oder Tandem APC-Cy7, primäre bzw. sekundäre Antikörper geeignet und kommerziell erhältlich (beispielsweise von dem Firmen Becton Dickinson, Dako, Coulter). Zum Nachweis polypeptidspezifischer Immunzellen eignen sich neben dem FACS Verfahren auch andere Verfahren, welche geeignet sind, die Produktion aus Immunzellen zu bestimmen, beispielsweise ELISA-Verfahren, Elispot Verfahren und Biosensoren. Diese Verfahren zur Bestimmung von Zytokinen sind vielfach beschrieben und Stand der Technik.

Das erfindungsgemäße Verfahren zum Nachweis polypeptidspezifischer T-Zellen ist geeignet für eine Vielzahl unterschiedlicher wissenschaftlicher und medizinischer Anwendungen, z.B. in der Analyse, Diagnostik und Therapie. Mit dem erfindungsgemäßen Verfahren können gleichzeitig verschiedene Populationen polypeptidspezifischer Immunzellen identifiziert werden z.B. zum Monitoring der Effizienz therapeutischer und prophylaktischer Impfungen zur Induktion von Immunzellen, zur Bestimmung der Effizient therapeutischer Behandlungen von Erkrankungen mit Beteiligung von Immunzellen, zum Screening der Sicherheit und Effizienz von Medikamenten, die eine Deletion oder Anergie von Immunzellen hervorrufen oder eine generelle Immunsuppression bewirken, zum Monitoring und der Diagnostik von durch Mikroorganismen und Parasiten induzierten Erkrankungen mit Immunzellbeteiligung, zum Monitoring und zur Diagnostik chronischer Entzündungen mit Immunzellbeteiligung, zum Monitoring und zur Diagnostik von Tumorantigen-spezifischen Immunzellen, zum Monitoring und zur Diagnostik von Immunzellen, die bei der Transplantatabstoßung eine Rolle spielen, zur Diagnostik von Autoimmunerkrankungen, oder zur gezielten Auswahl von Probanden für Impfstudien und die Testung therapeutischer Behandlungen.

Die erfindungsgemäßen Verfahren finden Anwendung bei allen Vertebraten, die Immunzellen, insbesondere T-Zellen besitzen, insbesondere am Menschen, Primaten und Nagetieren. Der Nachweis und die Quantifizierung polypeptidspezifischer Immunzellen kann beispielsweise aus Patienten, die an einer mikrobiellen Infektion, einer Tumorerkrankung, einer chronisch entzündlichen Erkrankung, einer Transplantatabstoßung oder einer Autoimmunerkrankung leiden, erfolgen, oder aber aus gesunden Probanden oder Teilnehmern therapeutischer oder präventiver Studien. Zudem kann der Nachweis von epitopspezifischen T-Zellen mittels mit dem erfindungsgemäßen Verfahren erhaltenen Zellen, vorzugsweise mittels erhaltener APCs auch in Primaten oder anderen Tieren erfolgen, die epitopspezifische Immunzellen besitzen.

Im Gegensatz zu den bislang beschriebenen Verfahren zum Nachweis von polypeptidspezifischen Immunzellen zeichnet sich das erfindungsgemäße Verfahren durch die folgenden Vorteile aus:
(1) Der Nachweis verschiedener Populationen polypeptidspezifischer Immunzellen kann gleichzeitig durchgeführt werden.
(2) Gleichzeitig kann die Quantität und Qualität verschiedener Populationen polypeptidspezifischer Immunzellen nachgewiesen werden.
(3) Das Verfahren kann einfach mittels kommerziell erhältlicher und in vielen Diagnostiklabors routinemäßig verwendeter Gerätschaften (FACS) durchgeführt werden.
(4) Das Verfahren kann unabhängig von dem Haplotyp des Probanden/Patienten universell für den Nachweis reaktiver polypeptidspezifischer Immunzellen eingesetzt werden.
(5) Das erfindungsgemäße Diagnostikverfahren eignet sich zur patientenspezifischen Bestimmung der Lymphozytenreaktivität gegen ein komplexes Polypeptid. Hierbei ist die Kenntnis von Zielepitopen dieser Immunzellen zur Durchführung des Verfahrens nicht erforderlich.
(6) Das Nachweisverfahren für polypeptidspezifische Immunzellen ist im Vergleich zu den herkömmlichen Diagnostikverfahren (CTL-Assay, ELISPOT, Zytokin-ELISA, Proliferationsassay) universell einsetzbar, leichter handhabbar, deutlich kostengünstiger, weniger zeitaufwendig und sensitiver.

Die Erfindung wird durch die nun folgenden Beispiele erläutert, ist aber nicht auf diese beschränkt:

### Beispiel 1: Produktion und Reinigung des Epstein Barr Virus (EBV) BZLF1 Polypeptides in E.coli.

Die cDNS für das BZLF1 Protein des EBV Stammes B95-8 wurde mittels PCR unter Verwendung geeigneter PCR-Bedingungen (Einleitender Denaturierungsschritt: 94° C, 2 Min; darauffolgend eine 3-Stufen PCR (15 Zyklen) mit folgenden Bedingungen: Denaturierung: 94° C, 30 Sek.; Annealing 52° C, 1 Min.; Elongation: 72° C, 2 Min; danach nochmals (25 Zyklen) mit folgenden Bedingungen: Denaturierung: 94° C, 30 Sek.; Annealing 62° C, 1 Min.; Elongation: 72° C, 2 Min; und einem abschließenden Polymerisationsschritt bei 72° C für 10 Min. mit anschließender Dauerkühlung bei 4° C) aus dem Plasmid pCMVZ (Manet *et al.* (1990); EMBO 8:1819) amplifiziert. Bei der PCR Reaktion zur Amplifikation des BZLF1 cDNS wurde dem PCR Ansatz 2,5% DMSO beigefügt. Zur Amplifikation wurden die Primer A (5' Primer: 5'-GGCGGAGATCYTTAGAAATTTAAGAGATCC-39; SEQ ID NO:1) und Primer B (3' Primer: 5'-GGCGGGGAATTCATGATGGACCCAAACTCG-3'; SEQ ID NO:2) verwendet. Die mittels der PCR amplifizierte Bande wurde dann mit den Restriktionsenzymen *Bgl*II und *Eco*RI gespalten und die in der Art erhaltene Bande in das ebenfalls mit *Bgl*ll und *Eco*RI linearisierte Plasmid pET (New England Biolabs) ligiert. Der so erzeugte Vektor wurde pET5c-Z genannt. Der bakterielle Expressionsvektor pET5c-Z wurde dann in den *E.coli* Stamm BL21-CodonPlus (DE3)-RIL (Carstens und Waesche (1999); Strategies; **12**:49) transformiert und die transformierten Bakterien für 1 Stunde bei 37°C auf LB Medium kultiviert. Danach wurden 50 µl der Kultur auf LB_{AMP} Platten ausgestrichen und diese über Nacht bei 37°C in einem Brutschrank kultiviert. Dann wurde eine Einzelkolonie in 200 ml LB Medium angeimpft und diese über Nacht bei 37°C bebrütet. Danach wurden 10 x 1 Liter LB Medium mit je 1/50 Volumen des Gesamtvolumens aus der Vorkultur angeimpft und die Kulturen anschließend bei 26°C bis zum erreichen einer O.D.₆₀₀ von 0,8 kultiviert. Anschließend wurden die Kulturen zur Stimulation der Proteinproduktion mit 1 mM IPTG versetzt und über Nacht weiter kultiviert.

Zur Zellemte wurde die Kultur 10 min. bei 5000 rpm in einem GS 3 Rotor in einer Sorvall Kühlzentrifuge zentrifugiert und das erhaltene Zellpellet in 250 ml Aufschlußpuffer (50 mM TrisCl pH 8,0 + 0,3M NaCl + 1 mM EDTA) resuspendiert. Danach wurde das resuspendierte Pellet bis zur weiteren Aufarbeitung bei -20°C weggefroren. Nach erneutem Auftauen des Zellpellets wurde der Zellsuspension zur Zelllyse eine Spatelspitze Lysozym (Muraminidase) zugegeben und der Ansatz für 20 min bei Raumtemperatur inkubiert. Danach wurden dem Ansatz 2 mM des Proteasehemmers Pefablock® zugegeben und dieser dann auf Eis einer Ultraschallbehandlung (3 x 1 min. bei Stufe 6, Pulsation: 80% mit einem Branson Sonifier, Standardspitze) unterzogen. Danach wurden dem Ansatz 5mM MgCl₂ und 2 U Benzonase®/ ml Proteinextrakt zugegeben, der Ansatz für 20 min bei 37°C inkubiert und anschließend (Schallsuspension), in einem GSA-Rotor für 30 min. bei 14000 rpm und 4°C zentrifugiert und der Überstand verworfen. Danach wurde das Pellet einer fraktionierenden Harnstoffwaschung unterzogen. Hierbei wurde das Pellet nach Ultraschallaufschluß in 250 ml 1M Harnstoff (in PBS ohne bivalente Ionen mit 2mM DTE) resuspendiert und die Suspension 1 Stunde in Überkopfschüttler bei 4°C inkubiert. Danach wurde die Suspension für 20 min. bei 14000 rpm zentrifugiert und der Überstand verworfen. Danach wurde das Pellet ein zweites mal, wie zuvor beschrieben, in 1M Harnstoff durch vortexen gelöst, die Suspension für 20 min. bei 14000 rpm zentrifugiert und der Überstand verworfen. Danach wurde das Pellet in 250 ml 2 M Harnstoff (in PBS ohne bivalente Ionen mit 2mM DTE) resuspendiert und die Suspension über Nacht in Überkopfschüttler bei 4°C inkubiert. Danach wurde die Suspension für 20 min. bei 14000 rpm zentrifugiert, das Pellet verworfen und der Überstand einer Säurefällung unterzogen. Dazu wurde der Überstand mit IN HCI auf pH 3,5 titriert und danach für 10 min bei 12000 rpm zentrifugiert. Das Pellet wurde verworfen und der Überstand 3 x gegen ein großes Volumen (3,5 Liter) Dialysepuffer (4M Harnstoff, 20 mM TrisC1 pH 7,5, 2mM DTE) dialysiert. Danach wurde die Suspension auf eine Poros QE Anionenaustauschersäule aufgetragen und die Säule mit 15 Säulenvolumina Laufpuffer (4M Harnstoff, 20mM TrisCl pH 7,5) gewaschen. Danach wurde das Protein mit 10 Säulenvolumina 8M Harnstoff, 2M NaCl eluiert und verschiedene Fraktionen aufgefangen. Anschließend wurden der Gehalt und die Reinheit des BZLF1 Proteins in verschiedenen Fraktionen mittels Coomassie- und Silberfärbung der mittels SDS-PAGE aufgetrennten Polypeptide, sowie durch Immunoblotting bestimmt. Fraktionen, welche das BZLF1 Protein mit hoher Reinheit aufweisen, wurden vereint und mit 2 mM DTE versetzt. Danach wurde die erhaltene BZLF1-haltige Suspension mittels einer Amikon-Rührzelle und einem YM10 Filter (Ausschlußvolumen 10 kDa) auf etwa 5 ml konzentriert und die Proteine in Suspension mittels Gelfiltationschromatographie weiter separiert. Hierzu wurde eine Superdex 200 prep grade, Pharmacia-HiLoad, 16/60. 120 ml CV, Flußrate: 0,5m1/min benutzt. Die Elution des BZLF1 Proteins erfolgte nach ca. 0,4 Säulenvolumen. Die sauberen BZLF1-Fraktionen wurden vereint und der Proteingehalt bestimmt. Mit diesem Verfahren kann das BZLF1 Protein mit einer Ausbeute von 3,9mg/1Liter Kultur und einer Reinheit von >95% gewonnen werden. Das BZLF1 Protein liegt nach der Reinigung mit einer Konzentration von 0,2 bis 1 mg/ml in 8 M Harnstoff, 2mM DTE vor.

### Beispiel 2: Nachweis von BZLF1-spezifischen zytotoxischen T-Zellen (CTL) aus peripherem Blut EBV-positiver Spender.

Die Austestung der Eignung Harnstoff-denaturierter und in Harnstofflösung vorliegender Polypeptide zur Induktion einer Epitoppräsentation auf MHC Klasse-I und -II Proteinen, sowie zum gleichzeitigen Nachweis polypeptidspezifischer CD4⁺ und CD8⁺ T-Zellen in gemischten APC/Lymphozytenkulturen wurde anhand eines sehr gut charakterisierten Modelsystems durchgeführt. Dieses beruht auf der Beobachtung, dass alle HLA B8-positiven, EBV-positiven Probanden CD8⁺ zytotoxische T-Zellen besitzen, die ein spezifisches Epitop (RAKFKQLL; Aminosäure 190-197; SEQ ID NO:3) innerhalb des EBV Proteins BZLF1 erkennen (Bogedain *et al.* (1995); J.Virol. 69:4872; Pepperl *et al.,* (1998) J.Virol. 72:8644). Zur Durchführung der Stimulationsexperimente wurden periphere Blutlymphozyten (PBMC) mittels Dichtegradientenzentrifugation aus heparinisiertem Vollblut (Heparin-Endkonzentration: 25 IU/ml) verschiedener EBV-negativer, HLA B8-positiver-, oder EBV-positiver, HLA B8-negativer bzw. EBV-positiver, HLA B8-positiver Probanden gewonnen. Hierfür wurde in speziellen 50 ml Leucosep-Röhrchen (Falcon, Becton Dickinson, Heidelberg) 15 ml Ficoll (PAN, Aidenbach) vorgelegt und mit Vollblut (2:1 mit PBS verdünnt) überschichtet. Bei der anschließenden Zentrifugation (30 min, 800 × g, Ausschwingrotor, Raumtemperatur) erfolgte eine Auftrennung in Plasma, Lymphozytenpopulation und Erythrozyten. Die gewünschte Lymphozytenpopulation wurde abgezogen und in ca. 30 ml PBS zweimal gewaschen. Die Sedimentation der Zellen erfolgte jeweils durch kurze Zentrifugation (5 min, 250 × g, Raumtemperatur). Das Zellpellet wurde abschließend in T-Zellmedium aufgenommen, die Zellzahl ermittelt und die Zellen entweder in den entsprechenden Versuchen eingesetzt.

Die Depletion von CD8⁺-Zellen aus frisch präparierten PBMC erfolgte durch negative Selektion mittels immunomagnetischer CD8 *MicroBeats* nach dem Protokoll des Herstellers (Miltenyi Biotec, Bergisch Gladbach). Frisch isolierte PBMC wurden gewaschen, zentrifugiert (300 × g, 10 min, 4 °C) und das Zellpellet in 80 µl PBS/10⁷ PBMC resuspendiert. Die magnetische Markierung der gewünschten Zellpopulation erfolgte durch Zugabe von 20 µl der spezifischen Microbeads/10⁷ PBMC während einer Inkubation von 15 min bei 4 °C. Anschließend wurde die Zellsuspension zweimal gewaschen (15 ml PBS), zentrifugiert und in 500 µl PBS aufgenommen. Für die magnetische Auftrennung wurden je nach Zellzahl MS⁺-Säulen (für maximal 1 × 10⁷ markierte Zellen) oder LS⁺-Säulen (für maximal 1 × 10⁸ markierte Zellen) verwendet, mit 5 ml PBS äquilibriert, die Zellsuspension aufgebracht und mit 3 × 500 µl (MS⁺-Säulen) bzw. 3 × 5ml (LS⁺-Säulen) PBS gewaschen. Nicht-markierte Zellen passieren die Säule. Anschließend wurde die Säule aus dem Magnetfeld entfernt und die markierte Zellpopulation durch Zugabe von 500 µl (MS⁺-Säulen) bzw. 5ml (LS⁺-Säulen) eluiert.

Der Nachweis von BZLF1-spezifischen CD4⁺ T-Helferzellen und CD8⁺ CTL erfolgte mittels *enzyme-linked immunospot assay* (Elispot). Mit Hilfe des Elispot können antigenspezifische T-Zellen anhand ihrer Zytokinsekretion (insbesondere der Sekretion von IFN-γ) detektiert werden. Nitrozellulosebeschichtete Mikrotiterplatten mit 96 Vertiefungen (MAHA S 45, Millipore, Eschborn) wurden mit monoklonalen, anti-human IFN-γ (Hölzl, Köln) beschichtet (5 µg/ml in PBS) und über Nacht bei 4 °C inkubiert. Die Antikörperlösung wurde abpipettiert und die Platten viermal mit je 200 µl PBS/Ansatz gewaschen. In einem anschließenden Schritt wurden unspezifische Bindungsstellen durch Zugabe von je 200 µl Blockierungspuffer (T-Zellmedium mit 10 % FKS) während einer einstündigen Inkubation bei 37°C blockiert. PBMC wurden in einer Konzentration von 2 × 10⁵ Zellen/Ansatz in einem Volumen von 100 µl T-Zellinedium/Ansatz (je 5 Replikate) eingesetzt. Die Stimulation erfolgte direkt in der Platte durch Zugabe von 50 µl Antigenlösung (Endkonzentration: BZLF1: 5 µg/ml). Nach einer 24stündigen Inkubation (37 °C, 5 % CO₂) wurde die Zellsuspension abgezogen und eventuell noch anhaftende Zellen durch 5maliges Waschen entfernt (je 200 µl PBS mit 0,05 % Tween 20, je 5 min Inkubation mit Waschpuffer). Danach erfolgte die Zugabe des sekundären, biotinylierten Antikörpers (1 µg/ml in PBS, je 100 µl/Ansatz); die Platten wurden für 2 Stunden bei 37 °C inkubiert, anschließend gewaschen (5 mal je 200 µl PBS/Ansatz) und für weitere 2 Stunden mit einem Streptavidin-Alkalische Phosphatase (AP)-Konjugat (1 µg/ml in PBS; je 200 µl/Ansatz; Hölzl, Köln) inkubiert. Nach einem letzten Waschschritt (5 mal je 200 µl PBS/Ansatz) erfolgte die Färbereaktion durch Zugabe des Enzymsubstrates NBT/BCIP (Boehringer, Mannheim). Dafür wurde eine Färbelösung [200 µl NBT/BCIP-Stammlösung auf 10 ml Färbepuffer (0,1 M Tris-Puffer, pH 9,5 mit 0,05 M MgCl₂, 0,1 M NaCl)] hergestellt und jeweils 200 µl/Ansatz eingesetzt. Nach 5-10 min (spenderspezifisch) wurde die kolorimetrische Reaktion durch Waschen der Platten mit deionisiertem Wasser gestoppt. Die Auswertung der Platten erfolgte an einem Elispot-Reader (Biosys 2000, BioSys, Karben).

Figur 1 zeigt die Ergebnisse des IFN-γ-Elispot am Beispiel von 8 Spendern (6 EBV-positive, 2 EBV-negative- Probanden) nach oben beschriebener Stimulation mit Harnstoff denaturiertem BZLF1 Polypeptid. PBMC der EBV-negativen (JW, BH) und/oder B8-negativen Spender (LD, FN, JW, BH) dienten als Negatikontrollen und zeigten keine signifikante Reaktivität nach antigene Stimulation mit Harnstoff-denarturiertem BZLF1. Im Gegensatz dazu konnte unter Verwendung von PBMC der EBV-positiven, HLA B8-positiven Spender (JU, SD, MB, RE) eine signifikante bis starke Reaktivität BZLF1-spezifischer IFN-γ produzierender Zellen gezeigt werden. Durch die Depletion der CD8⁺ T-Zellen reduzierte sich die Zahl detektierbarer IFN-γ produzierender signifikant, was das Vorhandensein BZLF1-spezifischer zytotoxischer T-Zellen in der Gesamtpopulation peripherer Blutlymphozyten belegt.

### Beispiel 3: Messung der Virus-spezifischen T-Lymphozyten in heparinisiertem Vollblut.

Einem serologisch positivem Spender werden 10 ml Vollblut in eine Heparin Monovette (Sarstedt, Nümbrecht) entnommen. Es werden davon je 2 ml in ein steriles Falcon Tube (Nr.2059; BD Falcon, Heidelberg) pipettiert. Ein Ansatz stellt die Negativ Kontrolle dar, hier werden zu dem Vollblut nur die puren monoklonalen Antikörper gegen die kostimulatorischen Moleküle CD28 und CD49d (Becton Dickinson, Heidelberg) in einer finalen Konzentration von 1 µg/ml hinzugegeben. Ein weiterer Ansatz ist die Positiv Kontrolle, hier wird zusätzlich zu den monoklonalen Antikörpern CD28 und CD49d ein Superantigen pipettiert. Es handelt sich dabei um das Staphylokokken Enterotoxin B (SEB), es wird in einer finalen Konzentration von 1 µg/ml hinzugeben. In einem dritten Ansatz wird zu dem Vollblut mit den monoklonalen Antikörpern noch das für die Restimulation verwendete Antigen hinzugegeben. Die Konzentration sollte für jede Charge neu austitriert werden. In unserem Fall konnten sowohl mit dem synthetischen Peptid als auch mit dem modifizierten Protein optimale Ergebnisse mit einer finalen Konzentration von 10 µg/ml erreicht werden. Die Röhrchen werden danach in stehend mit losen Deckel (der Gasautausch ermöglicht) in einem Standardbrutschrank (37°C, 5% CO₂ und H₂O-gesättigte Atmosphäre) inkubiert. Nach zwei Stunden wird zu den Proben Brefeldin A hinzugegeben, so daß eine finale Konzentration von 10 µg/ml erreicht wird. Die Proben werden auf einem Vortex gut durchmischt und für weitere 4 Stunden in den Brutschrank gestellt.

Nach insgesamt 6 Stunden Kurzzeitkultur werden 11 % des Kulturvolumens an eiskalter EDTA-Lösung (EDTA in PBS, 20mM) hinzupipettiert. Die Proben werden kurz gevortext und dann für maximal 10 Minuten bei Raumtemperatur inkubiert. Danach werden die Proben noch einmal gründlich gevortext, damit auch die adhärenten Zellen von der Röhrchenwand abgelöst werden. Zu den Proben wird dann mindestens das 9-fache des Kulturvolumens an FACS Lysing Solution (BD, Heidelberg) hinzugegeben. Die Röhrchen werden dann wieder für maximal 10 Minuten bei Raumtemperatur inkubiert. Anschließend werden die Proben bei 4°C für 8 Minuten mit 340g zentrifugiert. Der Überstand wird vorsichtig dekantiert und die Zellen werden dann mit 5 ml FACS-Puffer (PBS + 0.1 % w/v NaN₃ + 1% w/v FCS) gewaschen und wieder bei 340g für 8 Minuten zentrifugiert. Danach werden die Zellen in einer geringen Menge FACS-Puffer resuspendiert und die Zellen auf die Röhrchen (z.B. Falcon Nr.2054) zum Färben verteilt. Pro Färbeansatz sollten mindesten 1x10⁶ Zellen vorhanden sein. Dann wird zu den Zellen die Permeabilisienmgslöstmg (PBS + 0,1% w/v Saponin) in einer Menge von 1 ml pro 1x10⁶ Zellen pipettiert. Der Ansatz wird gut gevortext und für maximal 10 Minuten bei Raumtemperatur inkubiert. Die Zellen werden dann mit 5 ml FACS-Puffer gewaschen und bei 340g für 8 Minuten zentrifugiert. Danach können die Zellen gefärbt werden. Dies geschieht in einem Färbevolumen von 100 µl. Es werden die monoklonalen Antikörper in einem Verhältnis von FITC 1:10, PE 1:10, ECD 1:10 und APC 1:100 hinzupipettiert. Ein typisches 4-Farb-Röhrchen würde dann zum Beispiel die Antikörper CD3 FITC, anti-IFN-γ-PE, CD4 ECD und CD8 APC enthalten. Die Färbung erfolgt für mindestens 30 Minuten auf Eis (4°C) und in Dunkelheit. Die Zellen werden danach mindestens zweimal mit 5 ml FACS-Puffer gewaschen und nach jedem Waschschritt für 8 Minuten bei 340g zentrifugiert. Für die Messung werden die Zellen dann in mindestens 200 µl FACS Puffer aufgenommen. Und sofort an einem Durchflußzytometer analysiert. Sollten die Proben nicht gleich gemessen werden, können die Zellen mit 1% PFA (Paraformaldehyd) in FACS-Puffer fixiert werden. Die Lagerung erfolgt bei 4°C.

Die Ergebnisse werden dann mit Hilfe einer der CellQuest Software (Becton Dickinson, Heidelberg) ausgewertet. Die Abbildungen zeigen als Beispiel die Ergebnisse für einen EBVseropositiven Patienten der den HLA-Typ B8 aufweist. Nach der Permeabilisierung wurden folgende Färbungen durchgeführt:
- Ansatz 1: Kontrolle, ungefärbt
- Ansatz 2: CD3 FITC / IFN-γ PE / CD4 ECD / CD8 APC
- Ansatz 3: CD3 FITC / IL-4 PE / CD4 ECD / CD8 APC
- Ansatz 4: CD3 FITC / CD8 PE / CD4 ECD / CD69 APC
- Ansatz 5: CD3 FITC / CD16 PE / CD8 ECD / CD56 APC
- Ansatz 6: CD3 FITC / Perforin PE / CD8 ECD / CD4 APC
- Ansatz 7: CD3 FITC / Isotyp-Kontrolle PE / CD8 ECD / CD4 APC.

Figur 2a und b zeigen hierbei die durchflußzytometrischen Analysen von Vollblut nach oben beschriebener Restimulation mit synthetischem Peptid (Figur 2a) oder nach Restimulation mit Harnstoff denaturiertem BZLF1 Polypeptid (Figur 2b). Gezeigt ist ein FSC/SSC (Forward Scatter/Side Scatter) Dotplot, wobei der mit R1 (Region1) angegebene Messbereich der Lymphozytenpopulation entspricht. In der Figur 3a und b ist dann die Häufigkeit und Verteilung verschiedener Populationen CD3 und CD8-positiver Lymphozyten im Gesamtblut nach Stimulation mit Harnstoff denaturiertem BZLF1 Polypeptid (Figur 3a) oder einem synthetischen BZLF1 Peptid (Figur 3b), welches ein bekanntes CTL Epitop beinhaltet dargestellt. Hierbei wird deutlich, dass bei der Stimulation mit Harnstoff-adjuviertem Polypeptid deutlich mehr CD3 und CD8 schwach exprimierende Zellen, abgerufen werden können.

In Figur 4a und b ist die Häufigkeit der peripher vorkommenden doppelt positiven (CD4⁺ und CD8⁺) Lymphozyten Populationen im Gesamtblut nach Stimulation mit Harnstoff-adjuviertem BZLF1 Polypeptid (Figur 4a) oder einem synthetischen BZLF1 Peptid (Figur 4B), welches ein bekanntes CTL Epitop beinhaltet, dargestellt. Auch in dieser Abbildung werden deutlich die Vorzüge der Polypeptid-Restimulation deutlich, die eine erheblich bessere Analyse der doppelt positiven T Lymphozyten erlaubt, die gerade bei Virusinfektionen für das Immunsystem eine wichtige Rolle spielen können. Des weiteren sollte mit der Färbung der Oberflächenmarker CD16 und CD56 die Population der schwach CD3 und CD8 exprimierenden Zellen genauer charakterisiert werden. Denn sowohl bei CD16 als auch bei CD56 handelt es sich um Marker, die für NK bzw. NKT-Zellen typisch sind. Da die Zellen zusätzlich CD3 exprimieren, lassen sie sich damit den NKT Zellen zuordnen. Figur 5a und b zeigen in der Dotplot Darstellung Zellpopulationen welche die Expression der Oberflächenmarker CD8 und CD56 aufweisen. In beiden Figuren (Figur 5a und 5b) ist deutlich zu erkennen, dass die in Figur 2 mit R3 gekennzeichnete Population CD3⁺ CD8⁺ Zellen, hier in blau dargestellt, CD56 auf der Zelloberfläche exprimiert. Somit stellen die in R3 gezeigten Zellen die Population der NKT Zellen dar.

Des weiteren sollte mit dem Nachweis von intrazellulärem IFN-γ gezeigt werden, daß die T-Lymphozyten im Rahmen einer Th1 Immunantwort stimuliert worden sind und zytotoxische Eigenschaften besitzen. In den Figuren 7a und b ist in der Histogramm Plot Darstellung die Interferon-γ Expression aus verschiedenen Populationen der gesamten in R1 erfassten Lymphozyten nach Stimulation mit Harnstoff-adjuviertem BZLF1 Polypeptid (Figur 7a) oder dem BZLF1 Peptid (Figur 7b) gezeigt. M1 und M2 sind hier als Marker gesetzt. M1 bezeichnet den Bereich, in dem die IFN-γ Expression als positiv zu werten ist. Alles was links davon liegt, ist durch unspezifische Bindung des anti-IFN-γ Antikörpers innerhalb der Zellen zu erklären. Der durch M2 gekennzeichnete Bereich zeigt die IFN-γ Expression durch die reine Population CD3⁺CD8⁺ zytotoxischer T-Zellen. Die Differenz der in M1 und M2 dargestellten IFN-γ Werte kennzeichnet die IFN-γ Produktion durch schwach CD8⁺ Zellen mit NK-Zelleigenschaften. Nach Restimulation mit Harnstoff-adjuviertem BZLF1 Polypeptid (Figur 7a) kann im Vergleich zur Peptid-Stimulation eine deutlich erhöhte IFN-γ Produktion aus Zellen des Vollblutes sowie eine gesteigerte Stimulation der Population schwach CD8⁺ Zellen mit NK-Zelleigenschaften beobachtet werden. Diese Expression von intrazellulärem IFN-γ Gehalt wurde dann getrennt für die einzelnen Zellpopulationen ermittelt.

Figur 9a und b zeigen in der Histogramm Plot Darstellung die IFN-γ Expression der in R3 dargestellten Population CD8⁺ zytotoxischer T-Zellen aus Vollblut nach Stimulation mit Harnstoff-adjuviertem BZLF1 Polypeptid (Figur 9a) oder dem synthetischen BZLF1 Peptid (Figur 9b). Die Marker M1 und M2 sind entsprechend der Figur 7a und b gesetzt. Diese Abbildung zeigt, dass Harnstoff-adjuviertes BZLF1 Protein und das synthetische BZLF1 Peptid gleichermaßen zur Bestimmung BZLF1-spezifischer zytotoxischer T-Zellen geeignet sind. Das gleichzeitige Auslesen von CD8^{dim} Zellen war jedoch nur nach Behandlung der Zellen mit Harnstoff-adjuviertem BZLF1 Polypeptid möglich.

Deutlicher wird dieses Phänomen dann noch in der Figur 11a und b. Diese zeigen in der Histogramm Plot Darstellung die IFN-γ Expression der mit R4 benannten NKT-Zellpopulation nach Stimulation mit Harnstoff-adjuviertem BZLF1 Polypeptid (Figur 11a) oder dem synthetischen BZLF1 Peptid (Figur 11b). Diese Abbildung belegt die effiziente Stimulation der schwach CD8-positiven NKT-Zellpopulation nach Stimulation mit Harnstoff-adjuviertem BZLF1 Polypeptid.

Zusammenfassend läßt sich anhand der Beispiele zeigen, dass die dem bisherigen Stand der Technik entsprechende Methode, zytotoxische T-Lymphozyten mittels Restimulation mit synthetischen Peptiden auszulesen, tatsächlich nur eine Anaylse der CD8 hochpositiven T-Lymphozyten erlaubt. Bei dem Gebrauch von Harnstoff-adjuvierten Polypeptiden lassen sich allerdings wesentlich mehr Aussagen zu anderen Zellpopulationen (wie zum Beispiel CD4⁺CD8^{dim} Lymphozyten oder CD56⁺ NKT Zellen) machen.

### Beispiel 4: Induktion einer spezifischen Antikörperantwort in Kaninchen nach Immunisierung mit Harnstoff-adjuviertem BZLF1 Polypeptid.

Zur Austestung der Eignung Harnstoff-adjuvierter BZLF-1 Polypeptide wurden 30 µg BZLF1 Polypeptid in 8M Harnstoff nach Angaben des Herstellers (Sigma) mit Hunters Titermax adjuviert und einem Kaninchen intramuskulär verabreicht. 4 und 8 Wochen nach der Grundimmunisierung wurde das Tier mit dem selben Immunogen nachimmunisiert. Nach weiteren 3 Wochen wurde dem Tier Blut entnommen und das daraus gewonnene Serum in verschiedenen Verdünnungen (1:2000; 1:10000 und 1:50000) im Immunoblot auf die Anwesenheit BZLF1 spezifischer Antikörper getestet. Dazu wurde rekombinant in *E*. *coli* hergestelltes BZLF1 Polypeptid in verschiedenen Konzentrationen (20 ng, 100 ng und 500 ng) in einem 12,5%-igem SDS Gel separiert und die Proteine nachfolgend auf Nitrozelle transferiert. Nach dem Proteintransfer wurden die noch freien Proteinbindungsstellen auf der Nitrozellulose durch Inkubation mit einer 5%-igen Lösung von Magermilchpulver in TBS (500 mM NaCl, 25 mM Tris pH 7,5) unter leichtem Schütteln abgesättigt. Danach wurde der *Blot* viermal für mindestens 10 Min. mit TTBS gewaschen, bevor er für 1 bis 12 Std. mit der entsprechenden Verdünnung des Kaninchenserums in TBS inkubiert wurde.

### Detektion über eine Enzym/Substrat-vermittelte Farbreaktion:

Nach der Bindung des spezifischen Antikörpers wurde der Nitrocellulosefilter wiederum viermal für 10 Min. mit TTBS gewaschen und danach mindestens 1 Std. mit einer geeigneten Verdünnung eines mit alkalischer-Phosphatase (AP) bzw. Meerettich-Peroxidase (HRP) gekoppelten Anti-Immunglobulins geschüttelt. Nach einem weiteren Waschschritt (viermal 10 Min. mit TTBS) folgte die Inkubation des Filters mit den chromogenen Substraten der alkalischen Phosphatase (68 µl NBT, 70 µl BCIP in 20 ml AP-Puffer: 100 mM NaC1, 50 mM MgCl₂, 100 mM Tris (Sambrook *et al*. 1989)) bzw. Meerettich-Peroxidase (2,5 ml Tris/HCl pH 7,5, 1 Spatelspitze 3,3'-Diaminobenzidin, 30 µl 30 % H₂O₂, auf 50 ml mit H₂O_{bid.}). Die resultierenden Enzymreaktionen erzeugten nach Min. bis Std. eine braune Färbung. Die Reaktionen wurden jeweils mit H₂O_{bid}. abgestoppt.

Diese Untersuchungen ergaben, daß in 8 M Harnstoff gelöstes Polypeptid geeignet ist, um hohe Titer-polypeptidspezifischer Antikörper zu induzieren. So konnte gezeigt werden, daß das Serum des Versuchstieres nach 3 maliger Immunisierung noch in einer Verdünnung von 1:50000 genügend BZLF1 Antikörper enthält, um 100 ng gereinigten BZLF1 Polypeptids im Immunoblot zu detektieren (Fig. 12). Der in der Injektionslösung enthaltene Harnstoff erwies sich für das Versuchstier zudem als nicht toxisch.

### Beispiel 5: Einfluß von Harnstoff auf die Vitalität gereinigter Population peripherer blutmononukleärer Zellen (PBMC).

Zur Austestung des Einflusses von Harnstoff auf die Zellvitalität wurden PBMC, wie in Beispiel 2 beschrieben, aus dem heparinisierten Vollblut von 3 gesunden Spendern (DH, SB, IK) aufgereinigt und jeweils 1 x 10⁶ Zellen in vier Replikaten in T-Zellmedium (RPMI 1640 mit 5 % Humanserum, 2 mM Glutamin und 100 U/ml Penicillin/100 µg/ml Streptomycin (alle Komponenten von PanSystems, Aidenbach)) mit steigenden Konzentrationen Harnstoff (0, 0,05, 0,1, 0,2, 0,3, 0,4, 0,8 oder 1,6 mol/Liter) versetzt und in Vertiefungen einer 48-Loch-Mikrotiterplatte für 17 Stunden in einem Brutschrank mit befeuchteter Atmosphäre und 5% CO₂ Begasung bei 37°C inkubiert. Für diese Experimente wurde eine 8 M Harnstoffstocklösung verwendet, die mit 2M NaCl und 2mM DTE versetzt war. Danach wurden die Zellen in dem Kultivierungsansatz durch mehrmaliges auf- und abpipettieren des Mediums resuspendiert und die Gesamtzellzahl, sowie das Verhältnis der lebenden zu toten Zellen nach Färbung mit einer Trypanblau Lösung (0,5% w/v) mittels einer Neubauer _{"}Improved" Zählkammer bestimmt. Die Ergebnisse dieser Untersuchungen sind in Tabelle 1 dargestellt. Diese Untersuchungen ergaben, das der Zusatz von Harnstoff in einem Konzentrationsbereich von 0 bis 0,2 Mol/Liter im Vergleich zu unbehandelten Zellen zu keiner signifikanten Abnahme des Verhältnisses lebender zu toter Zellen in dem Gemisch peripherer mononukleärer Zellen führt. Bei Zugabe von 0,3 Mol/Liter Harnstoff war eine signifikant erhöhte Zahl toter Zellen zu beobachten; ab einer Harnstoffkonzentration von 0,4 Mol/Liter waren in den PBMC Kulturen nach 17 stündiger Inkubation, unabhängig von dem Spender, mehr tote als lebende Zellen vorhanden. Die Zugabe von Harnstoff in Konzentrationen ab 0,3 Mol/Liter führte zudem zu einer signifikanten Reduktion der Gesamtzellzahl, was auf einen zytolytischen Effekt von Harnstoff bei diesen Konzentrationen hindeutet.

**Tabelle: 1. Einfluss der Harnstoffkonzentration auf die Vitalität von PBMCs**

| C[Harnstoff] | D.H. | | | S.B. | | | I.K. | |
|---|---|---|---|---|---|---|---|---|
| | Zellzahl | Lebend / Tod | | Zellzahl | Lebend / Tod | | Zellzahl | Lebend / Tod |
| 0 Mol/L | 1,44 x 10⁶ | 0,5 | | 1,33 x 10⁶ | 34,4 | | 1,5 x 10⁶ | 121 |
| 0,05 Mol/L | 0,62 x 10⁶ | 15,9 | | 1,03 x 10⁶ | 2,2 | | 1,1 x 10⁶ | 11,7 |
| 0,1 Mol/L | 0,56 x 10⁶ | 1,7 | | 1,41 x 10⁶ | 55 | | 1,6 x 10⁶ | 41,3 |
| 0,2 Mol/L | 0,47 x 10⁶ | 6,6 | | 1,94 x 10⁶ | 3,6 | | 5,1 x 10⁶ | 30,2 |
| 0,3 Mol/L | n.d. | n.d. | | 0,36 x 10⁶ | 1,8 | | 0,7 x 10⁶ | 9 |
| 0,4 Mol/L | 0,02 x 10⁶ | 1 | | 0,48 x 10⁶ | 0,2 | | 0,3 x 10⁶ | 0,2 |
| 0,8 Mol/L | 0,05 x 10⁶ | 0,1 | | 0,35 x 10⁶ | 0,04 | | 2,9 x 10⁶ | 0,02 |
| 1,6 Mol/L | 0,91 x 10⁶ | 0,06 | | 0,76 x 10⁶ | 0 | | 0,4 x 10⁶ | 0 |

| | | | | | | | | |
|---|---|---|---|---|---|---|---|---|
| n.d.: nicht bestimmt; Lebend/TodVerhältnis = 0: das heißt, ausschließlich tote Zellen im Ansatz, bei unter 1 sind mehr tote als lebende Zellen im Ansatz. | | | | | | | | |

### Beispiel 6: Einfluß von Harnstoff auf die Zytokinsekretion aus gereinigten Population peripherer blutmononukleärer Zellen (PBMC).

Desweiteren sollte mittels ELIspot Analysen abgeklärt werden, ob Harnstoff in Populationen gereinigter PBMCs die Sekretion von Zytokinen, insbesondere IFN-γ auslöst. Dazu wurden nitrozellulosebeschichtete Mikrotiterplatten mit 96 Vertiefungen, wie in Beispiel 2 ausführlich beschrieben, mit einem monoklonalen, anti-human IFN-γ Antikörper (Hölzl, Köln) beschichtet (5 µg/ml in PBS) und über Nacht bei 4 °C inkubiert. Die Antikörperlösung wurde abpipettiert und die Platten viermal mit je 200 µl PBS/Ansatz gewaschen. In einem anschließenden Schritt wurden unspezifische Bindungsstellen durch Zugabe von je 200 µl Blockierungsmedium (RPMI mit 10 % FKS) während einer einstündigen Inkubation bei 37 °C blockiert. PBMC eines gesunden Spenders (SB) wurden in einer Konzentration von jeweils 2 x 10⁵ gereinigte PBMC/150 µL in T-Zellmedium enthaltend unterschiedliche Konzentrationen Harnstoff (0, 0,05, 0,1, 0,2, 0,3, 0,4, 0,8, und 1,6 Mol/Liter) (je 5 Replikate) aufgenommen und die Stimulationsansätze für 24 Stunden in einem Brutschrank mit befeuchteter Atmosphäre mit 5% CO₂ Begasung bei 37°C inkubiert. Für diese Experimente wurde eine 8 M Harnstoffstocklösung verwendet, die mit 2M NaCI und 2mM DTE versetzt war. Danach wurde die Zellsuspension abgezogen und eventuell noch anhaftende Zellen durch 6-maliges Waschen entfernt (je 200 µl PBS mit 0,1 % Tween 20, je 3 min Inkubation mit Waschpuffer). Danach erfolgte die Zugabe des sekundären, biotinylierten Antikörpers (1 µg/ml in PBS, je 100 µl/Ansatz); die Platten wurden für 2 Stunden bei RT inkubiert, anschließend gewaschen (6 mal je 200 µl PBS/Ansatz) und für 1 Stunde mit einem Streptavidin-Alkalische Phosphatase (AP)-Konjugat (1 µg/ml in PBS; je 100 µl/Ansatz; Hölzl, Köln) inkubiert. Nach einem letzten Waschschritt (6 mal je 200 µl PBS/Ansatz) erfolgte die Färbereaktion durch Zugabe des Enzymsubstrates NBT/BCIP (Boehringer, Mannheim). Dafür wurde eine Färbelösung [200 µl NBT/BCIP-Stammlösung auf 10 ml Färbepuffer (0,1 M Tris-Puffer, pH 9,5 mit 0,05 M MgCl₂, 0,1 M NaCl)] hergestellt und jeweils 100 µl/Ansatz eingesetzt. Nach 10 min wurde die kolorimetrische Reaktion durch Waschen der Platten mit entionisiertem Wasser gestoppt. Die Auswertung der Platten erfolgte an einem Elispot-Reader (Biosys 2000, BioSys, Karben).

Tabelle 2 zeigt die Ergebnisse des IFN-γ-Elispot am Beispiel eines Spenders nach Stimulation mit zuvor beschriebenen Harnstoffkonzentrationen. Diese Untersuchungen ergaben, dass nach Zugabe von Harnstoff unterschiedlicher Konzentration keine signifikant gesteigerte Freisetzung von IFN-γ aus Kulturen peripherer blutmononukleärer Zellen induziert wird.

**Tabelle: 2. Einfluss der Harnstoffkonzentration auf die IFN-γ Sekretion aus PBMCs**

| S.B. | | |
|---|---|---|
| c[Harnstoff] | Spots/2 x 10⁶ Zellen | Standardabweichung |
| 0 Mol/L | 18,4 | 5,46 |
| 0,05 Mol/L | 26,2 | 12,9 |
| 0,1 Mol/L | 29,6 | 9,99 |
| 0,2 Mol/L | 26,4 | 16,5 |
| 0,3 Mol/L | 11,0 | 4,15 |
| 0,4 Mol/L | 18,2 | 3,12 |
| 0,8 Mol/L | 14,6 | 3,56 |
| 1,6 Mol/L | 13,4 | 1,74 |

### Beispiel 7: Dosisabhängigkeit der IFN-γ Sekretion nach Stimulation mit Harnstoff behandeltem BZLF-1 Protein.

Die Austestung der Eignung Harnstoff-denaturierter und in Harnstofflösung vorliegender Polypeptide zur Induktion einer Epitoppräsentation auf MHC Klasse-I und -II Proteinen, sowie zum gleichzeitigen Nachweis polypeptidspezifischer CD4⁺ und CD8⁺ T-Zellen in gemischten APC/Lymphozytenkulturen wurde anhand eines sehr gut charakterisierten Modelsystems durchgeführt. Dieses beruht auf der Beobachtung, dass alle HLA B8-positiven, EBV-positiven Probanden CD8⁺ zytotoxische T-Zellen besitzen, die ein spezifisches Epitop (RAKFKQLL; Aminosäure 190-197) innerhalb des EBV Proteins BZLF1 erkennen (Bogedain *et al.* (1995); J.Virol. 69:4872; Pepperl *et al.,* (1998) J.Virol. 72:8644). Zur Austestung der optimalen Proteinkonzentration zur Detektion BZLF-1-spezifischer T-Zellantworten wurden gereinigte periphere Blutlymphozyten (PBMC) verschiedener EBV-negativer, HLA B8-positiver- (Spender 1), oder EBV-positiver, HLA B8-negativer (Spender 2,3) bzw. EBV-positiver, HLA B8-positiver Probanden (Spender 4-7) mit verschiedenen Konzentrationen Harnstoff-adjuvierter BZLF-1 Proteinen inkubiert und die IFN-γ Sekretion nach 17 Stunden mittels des ELIspot Verfahrens bestimmt.

Dazu wurden nitrozellulosebeschichtete Mikrotiterplatten mit 96 Vertiefungen, wie in Beispiel 2 ausführlich beschrieben, mit einem monoklonalen, anti-human IFN-γ Antikörper (Hölzl, Köln) beschichtet (5 µg/ml in PBS) und über Nacht bei 4 °C inkubiert. Die Antikörperlösung wurde abpipettiert und die Platten viermal mit je 200 µl PBS/Ansatz gewaschen. In einem anschließenden Schritt wurden unspezifische Bindungsstellen durch Zugabe von je 200 µl Blockierungsmedium (RPMI mit 10 % FKS) während einer einstündigen Inkubation bei 37 °C blockiert. Dann wurden PBMCs der sieben Probanden in einer Konzentration von jeweils 2 x 10⁵ gereinigten PBMC/150 µL in T-Zellmedium enthaltend unterschiedliche Konzentrationen Harnstoff-adjuviertem BZLF-1 Proteins (0,5 µg/ml, 2 µg/ml, 5 µg/ml, 20 µg/ml) (je 5 Replikate) aufgenommen und die Stimulationsansätze für 24 Stunden in einem Brutschrank mit befeuchteter Atmosphäre mit 5% CO₂ Begasung bei 37°C inkubiert. Für diese Experimente wurde BZLF-1 Protein verwendet, das mit einer Konzentration von 1mg/ml in einer 8 molaren Harnstoff mit 2M NaCl und 2mM DTE vorlag. Danach wurde die Zellsuspension abgezogen und eventuell noch anhaftende Zellen durch 6-maliges Waschen entfernt (je 200 µl PBS mit 0,1 % Tween 20, je 3 min Inkubation mit Waschpuffer). Danach erfolgte die Zugabe des sekundären, biotinylierten Antikörpers (1 µg/ml in PBS, je 100 µl/Ansatz); die Platten wurden für 2 Stunden bei RT inkubiert, anschließend gewaschen (6 mal je 200 µl PBS/Ansatz) und für 1 Stunde mit einem Streptavidin-Alkalische Phosphatase (AP)-Konjugat (1 µg/ml in PBS; je 100 µl/Ansatz; Hölzl, Köln) inkubiert. Nach einem letzten Waschschritt (6 mal je 200 µl PBS/Ansatz) erfolgte die Färbereaktion durch Zugabe des Enzymsubstrates NBT/BCIP (Boehringer, Mannheim). Dafür wurde eine Färbelösung [200 µl NBT/BCIP-Stammlösung auf 10 ml Färbepuffer (0,1 M Tris-Puffer, pH 9,5 mit 0,05 M MgCl₂, 0,1 M NaCl)] hergestellt und jeweils 100 µl/Ansatz eingesetzt. Nach 5-10 min (spenderspezifisch) wurde die kolorimetrische Reaktion durch Waschen der Platten mit entionisiertem Wasser gestoppt. Die Auswertung der Platten erfolgte an einem Elispot-Reader (Biosys 2000, BioSys, Karben).

**Tabelle 3: Relation der BZLF1 Proteinkonzentration zur Harnstoffkonzentration**

| c[BZLF1] | c[Hamstoff] |
|---|---|
| 5 µg/ml | 0,04 mol/Liter |
| 10 µg/ml | 0,08 mol/Liter |
| 20 µg/ml | 0,16 mol/Liter |
| 30 µg/ml | 0,24 mol/Liter |

Relation der BZLF1 Proteinkonzentration zur Harnstoffkonzentration bei Verwendung einer Stocklösung enthaltend 1 µg/µl BZLF1 Protein in 8 mol/Liter Harnstoff, 2 mol/Liter NaCl, 2 mmol/Liter 1,4-Dithioerythritol (DTE).

Die Ergebnisse dieses Experiments sind in der Figur 13 dargestellt. Die Untersuchungen zeigten, die BPMCs von 2 der 4 getesteten HLA-B8-positiven, EBV-positiven Spender bei allen getesteten BZLF-1 Konzentrationen eine im Vergleich zu den Negativkontrollen (Spender 1-3) deutlich gesteigerte Zahl IFN-γ produzierender BZLF-1-spezifischer T-Zellen aufwiesen. In diesen Experimenten waren bereits geringe Konzentrationen des Harnstoff-adjuvierten BZLF-1 Proteins ausreichend, um in 3 von 4 HLA-B8-positiven, EBV-positiven Spendern eine im Vergleich zu den Negativkontrollen (Spender 1-3) deutlich gesteigerte Zahl BZLF-1-spezifischer IFN-γ produzierender T-Zellen nachzuweisen. Die optimale Stimulation der Interferon-γ Produktion war Spender-spezifisch nach Stimulation mit 5 oder 20 µg/ml Harnstoff-adjuviertem BZLF-1 Protein zu beobachten. Nach Stimulation von PBMCs der Kontrollprobanden 1 und 3 mit Harnstoff-adjuviertem BZLF-1 Protein in Konzentrationen von 2 bis 20 µg/ml war eine geringe Zahl IFN-γ sezernierender PBMCs nachweisbar. Diese Reaktivitäten beruhen möglicherweise auf der Stimulation von CD8+ T-Zellen, die gegen bislang noch nicht bekannte Zielepitope innerhalb des BZLF-1 Proteins gerichtet sind. Desweiteren könnten die beobachteten Spots von BZLF-1-spezifischen CD4+ Zellen stammen, die durch die Stimulation mit dem Harnstoff-adjuvierten BZLF-1 Protein zur IFN-γ Produktion angeregt werden.

### Beispiel 8: Zeitlicher Verlauf der IFN-γ Sekretion nach Stimulation mit Harnstoff behandeltem BZLF-1 Protein.

Zur Austestung der optimalen Stimulationszeit zur Detektion BZLF-1-spezifischer T-Zellantworten wurden gereinigte periphere Blutlymphozyten (PBMC) verschiedener EBV-negativer, HLA B8-positiver- (Spender 1), oder EBV-positiver, HLA B8-negativer (Spender 2,3) bzw. EBV-positiver, HLA B8-positiver Probanden (Spender 4-7) unterschiedlich lange mit je 10 µg/ml Harnstoff-adjuvierter BZLF-1 Proteinen inkubiert und die IFN-γ Sekretion mittels des ELIspot Verfahrens bestimmt. Dazu wurden nitrozellulosebeschichtete Mikrotiterplatten mit 96 Vertiefungen, wie bereits ausführlich beschrieben, mit einem monoklonalen, anti-human IFN-γ Antikörper beschichtet und unspezifische Bindungsstellen durch Zugabe von Blockierungsmedium (RPMI mit 10 % FKS). Dann wurden PBMCs der sieben Probanden in einer Konzentration von jeweils 2 x 10⁵ gereinigten PBMC/150 µL in T-Zellmedium mit 10 µg/ml Harnstoff-adjuviertem BZLF-1 Protein (je 5 Replikate) aufgenommen und die Stimulationsansätze für 2, 8, 16 oder 24 Stunden in einem Brutschrank mit befeuchteter Atmosphäre mit 5% CO₂ Begasung bei 37°C inkubiert. Für diese Experimente wurde BZLF-1 Protein verwendet, das mit einer Konzentration von 1mg/ml in einer 8 molaren Harnstoff mit 2M NaCl und 2mM DTE vorlag. Die Durchführung des IFN-γ ELIspot Assays erfolgte, wie bereits ausführlich in dem vorhergehenden Beispiel beschrieben.

Die Ergebnisse dieses Experiments sind in der Figur 14 dargestellt. Die Untersuchungen zeigten, das bereits nach 8 Stunden Inkubation alle HLA-B8-positiven, EBV-positiven Spender (Spender 4-7) eine im Vergleich zu allen "Kontrollprobanden" (Spender 1-3) signifikant gesteigerte Zahl IFN-γ produzierender BZLF-1-spezifischer T-Zellen aufwiesen. Die maximale Zahl IFN-γ produzierender Zellen war spenderabhängig nach 16 bis 24 Stunden beobachtbar.

## Patentansprüche

1. Verfahren zum Polypeptidtransfer in Antigenfräsentierenden Zellen (APC-Zellen), umfassend die folgenden Schritte:
a) Inkubieren von Polypeptiden mit einer Harnstofflösung,
b) Inkubieren von Zellen mit den in der Harnstofflösung vorliegenden Polypeptiden.

2. Verfahren zum Nachweis polypeptidspezifischer Immunzellen, umfassend die folgenden Schritte:
a) Inkubieren von Polypeptiden mit einer Harnstofflösung,
b) Inkubieren von APC-haltigen Zellkulturen oder Körperflüssigkeiten mit den in der Harnstofflösung vorliegenden Polypeptiden,
c) Inkubieren der nach Schritt b) erhaltenen APC-haltigen Zellkulturen oder Körperflüssigkeiten mit Immunzellen oder immunzellhaltigen Körperflüssigkeiten,
d) gleichzeitiger Nachweis und/oder Quantifizierung verschiedener Subtypen von Immunzellen, die spezifisch gegen die Polypeptide aus Schritt a) sind.

3. Verfahren nach Anspruch 1 oder 2, wobei die Harnstofflösung in Schritt a) eine Konzentration im Bereich von etwa 0,01 mol/Liter bis etwa 10 mol/Liter aufweist.

4. Verfahren nach einem der Ansprüche 1 bis 3, wobei die Polypeptide in Schritt a) eine Konzentrationen im Bereich von etwa 0,01 µg/µl bis etwa 50 µg/µl aufweisen.

5. Verfahren nach einem der Ansprüche 1 bis 4, wobei die Polypeptide in Schritt b) bei etwa 10⁶ Zellen eine Konzentration im Bereich von etwa 0,1 µg bis etwa 200 µg aufweisen.

6. Verfahren nach einem der Ansprüche 1 bis 4, wobei die Harnstoffkonzentration in Schritt b) eine Endkonzentration im Bereich von etwa 0,001 bis etwa 0,3 mol/Liter oder weniger als 0,001 mol/Liter oder mehr als 0,3 mol/Liter aufweist.

7. Verfahren nach Anspruch 6, wobei die Harnstofflösung zusätzlich NaCl mit einer Konzentration im Bereich von etwa 0,25 mmol/Liter bis etwa 75 mmol/Liter und/oder DTE mit einer Konzentration im Bereich von etwa 0,25 nmol/Liter bis etwa 75 nmol/Liter enthält.

8. Verfahren nach einem der Ansprüche 2 bis 7, wobei die APC-haltige Zellkultur eine PBMC-Population (Leukapheresat), isolierte monozytäre Zellen oder eine separierte APC-Population und die APC-haltige Körperflüssigkeit Vollblut oder Liquor ist.

9. Verfahren nach Anspruch 8, wobei die separierte APC-Population dendritische Zellen (Langerhans Zellen), Monozyten, Makrophagen oder B-Zellen aufweist.

10. Verfahren nach einem der Ansprüche 2 bis 9, wobei die Immunzellen T-Zellen oder andere immunologische Zellpopulationen oder ein Gemisch davon sind und die immunzellhaltigen Körperflüssigkeiten Vollblut oder Liquor sind.

11. Verfahren nach Anspruch 10, wobei die T-Zellen CD4⁺ T-Zellen, CD8⁺ T-Zellen, CD4⁺CD8^{dim} T-Zellen oder CD4⁺CD25⁺ Suppressor T-Zellen sind und die immunologischen Zellpopulationen CD56⁺CD8⁺, CD56⁻CD57⁺CD8⁺ NKT-Zellen oder CD56⁺ NK-Zellen sind.

12. Verfahren nach einem der Ansprüche 2 bis 11, wobei der Nachweis und/oder die Quantifizierung mittels eines Nachweises von spezifischen Oberflächenmarkern für Immunzellen und IFNγ, IL4 oder IL5 durchgeführt wird.

13. Verfahren nach einem der Ansprüche 2 bis 12, wobei der Nachweis mittels FACS-, ELISA- oder Elispot-Verfahren oder Biosensoren durchgeführt wird.

## Claims

1. A method for polypeptide transfer in antigen presenting cells (APC cells), comprising the following steps:
a) Incubating polypeptides with a urea solution,
b) Incubating cells with the polypeptides present in the urea solution.

2. A method for the detection of polypeptide-specific immune cells, comprising the following steps:
a) Incubating polypeptides with a urea solution,
b) Incubating APC-containing cell cultures or body fluids with the polypeptides present in the urea solution,
c) Incubating the APC-containing cell cultures or body fluids obtained according to step b) with immune cells or immune-cell-containing body fluids,
d) Simultaneously detecting and/or quantifying various subtypes of immune cells which are specific against the polypeptides from step a).

3. The method according to claim 1 or claim 2, wherein the urea solution in step a) has a concentration in the range of about 0.01 mol/litre to about 10 mol/litre.

4. The method according to any one of claims 1 to 3, wherein the polypeptides in step a) have a concentration in the range of about 0.01 µg/µl to about 50 µg/µl.

5. The method according to any one of claims 1 to 4, wherein the polypeptides in step b) have a concentration in the range of about 0.1 µg to about 200 µg for approximately 10⁶ cells.

6. The method according to any one of claims 1 to 4, wherein the urea concentration in step b) has a final concentration in the range of about 0.001 to about 0.3 mol/litre or less than 0.001 mol/litre or more than 0.3 mol/litre.

7. The method according to claim 6, wherein the urea solution additionally contains NaCl with a concentration in the range of about 0.25 mmol/litre to about 75 mmol/litre and/or DTE with a concentration in the range of about 0.25 nmol/litre to about 75 nmol/litre.

8. The method according to any one of claims 2 to 7, wherein the APC-containing cell culture is a PBMC population (leukapheresate), isolated monocytic cells or a separated APC population and the APC-containing body fluid is whole blood or liquor.

9. The method according to claim 8, wherein the separated APC populations have dendritic cells (Langerhans cells), monocytes, macrophages or B cells.

10. The method according to any one of claims 2 to 9, wherein the immune cells are T cells or other immunological cell populations or a mixture thereof and the immune-cell-containing body fluids are whole blood or liquor.

11. The method according to claim 10, wherein the T cells are CD4⁺ T cells, CD8⁺ T cells, CD4⁺CD8^{dim} T cells or CD4⁺CD25⁺ suppressor T cells and the immunological cell populations are CD56⁺CD8⁺, CD56⁻CD57⁺CD8⁺ NKT cells or CD56⁺ NK cells.

12. The method according to any one of claims 2 to 11, wherein the detection and/or the quantification is carried out by means of a detection of specific surface markers for immune cells and IFNγ, IL4 or IL5.

13. The method according to any one of claims 2 to 12, wherein the detection is carried out using FACS, ELISA or Elispot methods or biosensors.

## Revendications

1. Procédé pour le transfert de polypeptides dans des cellules présentatrices d'antigènes (cellules APC), comportant les étapes suivantes :
a) mise en incubation de polypeptides avec une solution uréique,
b) mise en incubation de cellules avec les polypeptides présents dans la solution uréique.

2. Procédé pour détecter des cellules immunitaires spécifiques de polypeptides, comportant les étapes suivantes :
a) mise en incubation de polypeptides avec une solution uréique,
b) mise en incubation de cultures cellulaires ou de fluides corporels contenant des APC avec les polypeptides présents dans la solution uréique,
c) mise en incubation selon l'étape b) de cultures cellulaires ou de fluides corporels contenant des APC avec des cellules immunitaires ou des fluides corporels contenant des cellules immunitaires,
d) détection et/ou quantification simultanée de divers sous-types de cellules immunitaires spécifiquement dirigées contre les polypeptides résultant de l'étape a).

3. Procédé selon la revendication 1 ou 2, dans lequel la solution uréique de l'étape a) présente une concentration allant de 0,01 mol/litre environ à 10 mol/litre environ.

4. Procédé selon l'une des revendications 1 à 3, dans lequel les polypeptides de l'étape a) présentent une concentration allant de 0,01 µg/µl environ à 50 µg/µl environ.

5. Procédé selon l'une des revendications 1 à 4, dans lequel les polypeptides de l'étape b) présentent pour 10⁶ cellules environ une concentration allant de 0,1 µg environ à 200 µg environ.

6. Procédé selon l'une des revendications 1 à 4, dans lequel la concentration uréique de l'étape b) présente une concentration finale allant de 0,001 environ à 0,3 mol/litre environ ou de moins de 0,001 mol/litre ou de plus de 0,3 mol/litre.

7. Procédé selon la revendication 6, dans lequel la solution uréique contient en plus du NaCI avec une concentration allant de 0,25 mmol/litre environ à 75 mmol/litre environ et/ou du DTE avec une concentration allant de 0,25 nmol/litre environ à 75 nmol/litre environ.

8. Procédé selon l'une des revendications 2 à 7, dans lequel la culture cellulaire contenant des APC est une population de PBMC (leucophérèse), des cellules monocytaires isolées ou une population d'APC séparée, et le fluide corporel contenant des APC est du sang complet ou un soluté aqueux.

9. Procédé selon la revendication 8, dans lequel la population d'APC séparée présente des cellules dendritiques (cellules de Langerhans), des monocytes, des macrophages ou des cellules B.

10. Procédé selon l'une des revendications 2 à 9, dans lequel les cellules immunitaires sont des cellules T ou d'autres populations de cellules immunologiques ou un mélange des deux, et les fluides corporels contenant des cellules immunitaires sont du sang complet ou un soluté aqueux.

11. Procédé selon la revendication 10, dans lequel les cellules T sont des cellules T CD4⁺, des cellules T CD8⁺, des cellules T CD4⁺CD8^{dim} ou des cellules T suppressives CD4⁺CD25⁺, et les populations cellulaires immunologiques sont des cellules NKT CD56⁺CD8⁺, CD56⁻CD57⁺CD8⁺ ou des cellules NK CD56⁺.

12. Procédé selon l'une des revendications 2 à 11, dans lequel la détection et/ou la quantification sont effectuées au moyen d'une détection de marqueurs de surface spécifiques des cellules immunitaires et de IFNγ, IL4 ou IL5.

13. Procédé selon l'une des revendications 2 à 12, dans lequel la détection est effectuée au moyen d'un procédé FACS, ELISA ou Elispot ou de biocapteurs.
